# EUROPEAN PATENT APPLICATION

(11) **EP 3 868 887 A1**
(43) Date of publication of application: **25.08.2021**
(21) Application number: 19867133.1
(22) Date of filing: 24.09.2019
(51) Int. Cl.: C12N 15/861, C12N 15/09, C12N 15/63, C12N 15/863, C12N 15/866, C12N 15/867, C12N 15/869

(54) **NOVEL VIRUS VECTOR AND METHODS FOR PRODUCING AND USING SAME**

(30) Priority: 25.09.2018 JP 2018179274; 28.06.2019 JP 2019121668
(71) Applicant: Microbial Chemistry Research Foundation, Tokyo 141-0021 (JP)
(72) Inventor: SAITO, Izumu, Tokyo 141-0021 (JP); NAKANISHI, Tomoko, Tokyo 141-0021 (JP)
(74) Representative: Graf von Stosch Patentanwaltsgesellschaft mbH
(86) International application number: PCT/JP2019/037255
(87) International publication number: WO 2020/067004

(57) **Abstract**

The present invention relates to: a novel adenovirus vector having a shortened backbone; a nucleic acid vector expressing five or more Cas guide RNA; a nucleic acid vector containing a Cas protein-coding gene and a Cas guide RNA expression unit; a composition for genome editing containing these vectors; and a gene therapeutic method using these.

SEQUENCE LISTING

<110>  Microbial Chemistry Research Foundation

<120>  NEW VIRUS VECTOR, AND METHOD FOR PRODUCING THE SAME AND USE OF
       THE SAME

<130>  N-BK001-19P

<150>  JP 2018-179274
<151>  2018-09-25

<150>  JP 2019-121668
<151>  2019-06-28

<160>  52

<170>  PatentIn version 3.5

<210>  1
<211>  20
<212>  DNA
<213>  Artificial Sequence

<220>
<223>  Guide coding DNA

<400>  1
gcggcatcct ggtctctggg                                                   20


<210>  2
<211>  20
<212>  DNA
<213>  Artificial Sequence

<220>
<223>  Guide coding DNA

<400>  2
gcagcagagc tctgattctg                                                   20


<210>  3
<211>  19
<212>  DNA
<213>  Artificial Sequence

<220>
<223>  Guide coding DNA

<400>  3
gaggctgaga tggtggtca                                                    19


<210>  4
<211>  20
<212>  DNA
<213>  Artificial Sequence

<220>
<223>  Guide coding DNA

<400>  4
ggaggtgaag acgacattga                                                   20


<210>  5
<211>  20
<212>  DNA
<213>  Artificial Sequence

<220>
<223>  Guide coding DNA

<400>  5
gaagcgaagt gcacacggtc                                                   20


<210>  6
<211>  20
<212>  DNA
<213>  Artificial Sequence

<220>
<223>  Guide coding DNA

<400>  6
gaggtgaagc gaagtgcaca                                                   20


<210>  7
<211>  20
<212>  DNA
<213>  Artificial Sequence

<220>
<223>  Guide coding DNA

<400>  7
ggtttccatg cgacgtgcag                                                   20


<210>  8
<211>  20
<212>  DNA
<213>  Artificial Sequence

<220>
<223>  Guide coding DNA

<400>  8
ggcagatgag aaggcacaga                                                   20


<210>  9
<211>  20
<212>  DNA
<213>  Artificial Sequence

<220>
<223>  Guide coding DNA

<400>  9
gaagcgaagt gcacacggtc                                                   20

<210>  10
<211>  20
<212>  DNA
<213>  Artificial Sequence

<220>
<223>  Guide coding DNA

<400>  10
gaggtgaagc gaagtgcaca                                                   20


<210>  11
<211>  20
<212>  DNA
<213>  Artificial Sequence

<220>
<223>  Guide coding DNA

<400>  11
ggtttccatg cgacgtgcag                                                   20


<210>  12
<211>  20
<212>  DNA
<213>  Artificial Sequence

<220>
<223>  Guide coding DNA

<400>  12
gacctggtgg gcgttcacgg                                                   20


<210>  13
<211>  30
<212>  DNA
<213>  Artificial Sequence

<220>
<223>  Primer

<400>  13
aactggatcc tgcgcgggac gtcctttgtc                                        30


<210>  14
<211>  32
<212>  DNA
<213>  Artificial Sequence

<220>
<223>  Primer

<400>  14
gaaaaagatc tcagtggtat ttgtgagcca gg                                     32


<210>  15
<211>  20

<212>  DNA
<213>  Artificial Sequence

<220>
<223>  Guide coding DNA

<400>  15
gacctggtgg gcgttcacgg                                                   20


<210>  16
<211>  20
<212>  DNA
<213>  Artificial Sequence

<220>
<223>  Guide coding DNA

<400>  16
gtcttatata agaggactct                                                   20


<210>  17
<211>  20
<212>  DNA
<213>  Artificial Sequence

<220>
<223>  Guide coding DNA

<400>  17
gacatgaaca tgagatgatt                                                   20


<210>  18
<211>  20
<212>  DNA
<213>  Artificial Sequence

<220>
<223>  Guide coding DNA

<400>  18
gctgtgcctt gggtggcttt                                                   20


<210>  19
<211>  20
<212>  DNA
<213>  Artificial Sequence

<220>
<223>  Guide coding DNA

<400>  19
gattgagacc ttcgtctgcg                                                   20


<210>  20
<211>  20
<212>  DNA
<213>  Artificial Sequence

<220>
<223>  Guide coding DNA

<400>  20
gtcgcagaag atctcaatct                                                   20


<210>  21
<211>  20
<212>  DNA
<213>  Artificial Sequence

<220>
<223>  Guide coding DNA

<400>  21
gatatgggtg aggcagtagt                                                   20


<210>  22
<211>  20
<212>  DNA
<213>  Artificial Sequence

<220>
<223>  Guide coding DNA

<400>  22
gtcaatcttc tcgaggactg                                                   20


<210>  23
<211>  25
<212>  DNA
<213>  Artificial Sequence

<220>
<223>  Primer

<400>  23
ttaacttcat gggatatgta attgg                                             25


<210>  24
<211>  25
<212>  DNA
<213>  Artificial Sequence

<220>
<223>  Primer

<400>  24
actcaagatg ttgtacagac ttggc                                             25


<210>  25
<211>  33
<212>  DNA
<213>  Artificial Sequence

<220>
<223>  Primer

<400>  25
ttggatccaa gtctgcagct ggcaactttg acg                                    33


<210>  26
<211>  35
<212>  DNA
<213>  Artificial Sequence

<220>
<223>  Primer

<400>  26
aaagaattct acactacagg tacaaagggc ttcag                                  35


<210>  27
<211>  37
<212>  DNA
<213>  Artificial Sequence

<220>
<223>  Primer

<400>  27
gcgtgtacag tatatgcaaa tatgaaggaa tcatggg                                37


<210>  28
<211>  37
<212>  DNA
<213>  Artificial Sequence

<220>
<223>  Primer

<400>  28
gcgtgtacaa aatggactat catatgctta ccgtaac                                37


<210>  29
<211>  20
<212>  DNA
<213>  Artificial Sequence

<220>
<223>  Target sequence

<400>  29
gcggcaggga gaaattgaag                                                   20


<210>  30
<211>  20
<212>  DNA
<213>  Artificial Sequence

<220>
<223>  Target sequence

<400>  30
gccgcctggc tttggccacc                                                   20

<210>  31
<211>  20
<212>  DNA
<213>  Artificial Sequence

<220>
<223>  Target sequence

<400>  31
gatgaaagac cttgcccaga                                                    20


<210>  32
<211>  20
<212>  DNA
<213>  Artificial Sequence

<220>
<223>  Target sequence

<400>  32
gctctggcca tcctcatctg                                                    20


<210>  33
<211>  20
<212>  DNA
<213>  Artificial Sequence

<220>
<223>  Target sequence

<400>  33
gataccgtac tgggccacta                                                    20


<210>  34
<211>  20
<212>  DNA
<213>  Artificial Sequence

<220>
<223>  Target sequence

<400>  34
gcccctcagt gctttccttc                                                    20


<210>  35
<211>  20
<212>  DNA
<213>  Artificial Sequence

<220>
<223>  Target sequence

<400>  35
gaggttcatg tccccccttca                                                    20


<210>  36
<211>  20

<212>  DNA
<213>  Artificial Sequence

<220>
<223>  Target sequence

<400>  36
ggaccgaggg ttaagaggaa                                                   20


<210>  37
<211>  30
<212>  DNA
<213>  Artificial Sequence

<220>
<223>  Primer

<400>  37
gtcatggaca cagcctctcc tgaagacagc                                        30


<210>  38
<211>  30
<212>  DNA
<213>  Artificial Sequence

<220>
<223>  Primer

<400>  38
cctttacagt gatcctgtag agacttctgg                                        30


<210>  39
<211>  20
<212>  DNA
<213>  Artificial Sequence

<220>
<223>  Guide coding DNA

<400>  39
gcagaggtga aaaagttgca                                                   20


<210>  40
<211>  20
<212>  DNA
<213>  Artificial Sequence

<220>
<223>  Guide coding DNA

<400>  40
gacatgaaca tgagatgatt                                                   20


<210>  41
<211>  20
<212>  DNA
<213>  Artificial Sequence

<220>
<223>  Guide coding DNA

<400>  41
gctgtgcctt gggtggcttt                                                   20


<210>  42
<211>  20
<212>  DNA
<213>  Artificial Sequence

<220>
<223>  Guide coding DNA

<400>  42
gaagctccaa attctttata                                                   20


<210>  43
<211>  20
<212>  DNA
<213>  Artificial Sequence

<220>
<223>  Guide coding DNA

<400>  43
agacaaagga cgtcccgcgc                                                   20


<210>  44
<211>  20
<212>  DNA
<213>  Artificial Sequence

<220>
<223>  Guide coding DNA

<400>  44
gacccgtctc ggggccgttt                                                   20


<210>  45
<211>  20
<212>  DNA
<213>  Artificial Sequence

<220>
<223>  Guide coding DNA

<400>  45
gccggaacgg cagatgaaga                                                   20


<210>  46
<211>  20
<212>  DNA
<213>  Artificial Sequence

<220>
<223>  Guide coding DNA

<400>  46
ggggcgcacc tctctttacg                                                   20


<210>  47
<211>  20
<212>  DNA
<213>  Artificial Sequence

<220>
<223>  Guide coding DNA

<400>  47
ggtctccatg cgacgtgcag                                                   20


<210>  48
<211>  20
<212>  DNA
<213>  Artificial Sequence

<220>
<223>  Guide coding DNA

<400>  48
gaccaccgtg aacgcccacc                                                   20


<210>  49
<211>  20
<212>  DNA
<213>  Artificial Sequence

<220>
<223>  Guide coding DNA

<400>  49
gtcctcttat gtaagacctt                                                   20


<210>  50
<211>  20
<212>  DNA
<213>  Artificial Sequence

<220>
<223>  Guide coding DNA

<400>  50
gatgtcaacg accgaccttg                                                   20


<210>  51
<211>  31
<212>  DNA
<213>  Artificial Sequence

<220>
<223>  Primer

<400>  51
atcttatcat gtctggatca aatccgaacg c                                      31

<210>  52
<211>  86
<212>  DNA
<213>  Artificial Sequence

<220>
<223>  tracrRNA coding DNA

<400>  52
gttggaacca ttcaaaacag catagcaagt taaaataggc tagtccgtta tcaacttgaa     60

aaagtggcac cgagtcggtg cttttt                                          86

## Description

### Technical Field

The present invention relates to: a novel adenovirus vector having a shortened backbone; a nucleic acid vector containing five or more Cas guide RNA expression units; a nucleic acid vector containing a Cas protein-coding gene and a Cas guide RNA expression unit; a composition for genome editing containing these vectors; and a gene therapeutic method using these.

### Background Art

A CRISPR/Cas9 system, which is one of the CRISPR/Cas systems of prokaryotes, has been expected to be promising for clinical applications such as treatments of cancer and genetic diseases as a genome editing technology that efficiently re-writes the gene sequence (Non-Patent Literature 1). A basic CRISPR/Cas9 system is composed of: a Cas9 protein that cuts a DNA double strand; and guide RNA (gRNA). The gRNA is composed of: CRISPR RNA (crRNA) to determine the site specificity on the target genome; and activating RNA (trans-activating crRNA: tracrRNA), which contains partial complementarity to the crRNA and serves as a scaffold for binding to the Cas9 protein. The guide RNA composed of crRNA and tracrRNA can also be provided as single guide RNA (sgRNA) where these are fused together. This guide RNA can express in the target cells using an appropriate vector containing DNA encoding this (Non-Patent Literature 1).

The CRISPR/Cas9 system causes a site-specific genome cutting but its accuracy is not complete. In theory, the CRISPR/Cas9 system is expected to specifically cut a genome site having a 20-base sequence designated by the guide RNA. In actual, however, the CRISPR/Cas9 system exhibits specificity of only about 12 bases. As a result, the CRISPR/Cas9 system may cut the genome at positions other than those in the originally intended genome regions (off-target cutting) to cause off-target mutations due to this cutting. The human genome has a huge size of about 3000 M bases. Therefore, when the CRISPR/Cas9 system is directly applied to genome editing therapy for human, several hundreds of sites other than the target sites may be cut, which makes it impossible for such genome editing therapy to be practically used as a therapeutic method. In order to suppress unintended cuttings other than at the target sites to practically use the genome editing therapy for human, there is a need to considerably reduce the frequency of off-target mutations (Non-Patent Literature 2).

So far, various studies have been made on suppression of off-target mutations by the Cas9 protein. In one attempted study for considerably reducing the frequency of off-target mutations, the sequence of the guide RNA is adjusted or the amino acid sequence of the Cas9 protein is mutated to form Cas9 nickase that cuts only one strand of DNA (inserts a nick). At proximal positions (e.g., within 30 base pairs from the 5' ends of two guide RNA), the resultant Cas9 nickase forms nicks in the sense strand and the anti-sense strand of the double-stranded DNA (cutting of one strand). Only when the nicks are formed at the two positions, the genome is cut at the sites (double-nicking system) (Non-Patent Literature 2).

Use of the above double-nicking system can substantially make sure a sequence specificity of 24 bases long. It is expected that there will be almost zero in the frequency of off-target mutations presumable in the case of use in human genome editing. As a result, "safety" of genome editing therapy increases remarkably.

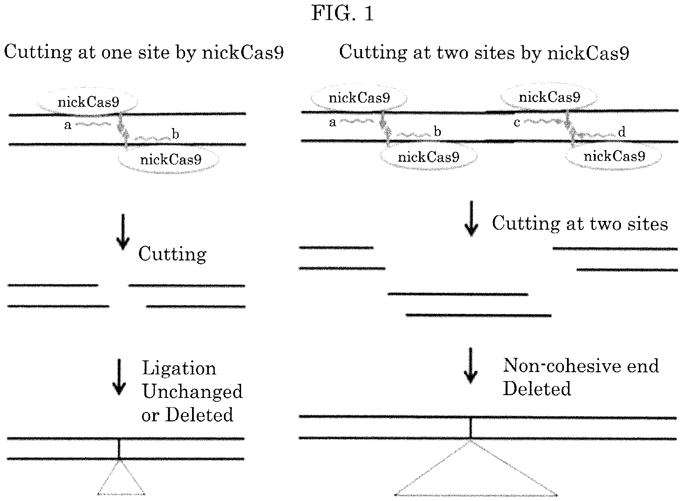

In the usual CRISPR/Cas9 system, the sense strand and the anti-sense strand of a double strand are cut at the same position to form blunt ends, and some of the cut sites are re-bonded by the DNA repair functions of cells. Meanwhile, when the above double-nicking system is configured to form 5' overhangs at the cut sites, the sense strand and the anti-sense strand at both ends of the cut sites are provided with 5' overhangs of several tens of bases (FIG. 1). This suppresses rebonding of the cut sites by the DNA repair functions of cells, which makes sure to irreversibly delete and destroy the target from the genome. Also, use of the 5' overhangs makes it possible to insert (knock-in) a foreign DNA fragment with high efficiency. Therefore, the double-nicking method remarkably increases "efficiency" of genome editing therapy.

That is, the double-nicking system makes genome editing therapy "safer" and "highly efficient".

Regardless of such improvements, however, there is a demand for a technology for making genome editing therapy much "safer" and further "highly efficient".

Here, the double-nicking system has to use two guide RNA (a guide RNA pair) to create one DNA double strand-cut site on the genome. In order to cut both ends of one DNA region from the genome to delete the region, two guide RNA pairs; i.e., four guide RNA are necessary (FIG. 1). It is known, however, that there is difficulty in simultaneously expressing two or more guide RNA from a single nucleic acid vector.

Another developing method is knocking down a gene expression by using a Cas9 mutant that is allowed to completely lose the ability to cut or form a nick (dead Cas9) (Non-Patent Literature 3). A vector that simultaneously expresses many guide RNA can simultaneously knock down expressions of many genes.
Such a technology has been demanded in basic research.

Without being bound by theory, it is believed that one reason for such difficulty is because the guide RNA expression units on the vector each contain an expression regulating unit having the same DNA sequence and thus without matching all the transcription directions of the guide RNA expression units, palindrome sequences will be formed between the adjacent expression units that are to be transcribed in different directions, and as a result, formation of DNA complementary strands within the palindrome sequences inhibits replication of the vector.

Another conceivable reason is because of missing of the guide RNA expression units as a result of homologous recombination between the above expression regulating units having the same DNA sequence by the action of DNA recombination functions of host cells. In particular, the COS-TPC (Non-Patent Literature 4 and Patent Literature 1), which has traditionally been used as a highly efficient production method for an adenovirus vector, uses homologous recombination in the production process. This is why it is believed that there has been difficulty in producing an adenovirus vector having many guide RNA expression units.

Recombinant adenoviruses have been used as an excellent DNA vector for introducing genes into animal cells because of their advantages such as high efficiency of gene introduction, the ability to introduce genes into non-dividing cells as well, and easiness in preparation of high-titer virus liquid. In addition, they have widely been used because of their recognized usefulness in not only gene therapy but also in, for example, analyses of gene functions in basic fields (Non-Patent Literatures 5 and 6).

There is the upper limit to the size of an adenovirus vector that is capable of replicating as a virus. Therefore, in order to further increase the size of a gene fragment to be delivered as a payload by an adenovirus vector, it is desirable to shorten a portion of the adenovirus vector other than the payload; i.e., the backbone structure, as much as possible. To this end, various attempts have been made to shorten the backbone structure of the adenovirus vector (Non-Patent Literature 6).

For example, traditionally used vectors derived from adenoviruses lack the E3 region that is not essential for virus replication. A known pBHG10 plasmid vector lacks a region of 2685 bases long from the E3 region.

An Ad5 vector further lacks the E1 gene region necessary for the expression of a virus gene and can only replicate in host cells such as human embryonic kidney-derived cell lines (293 cells) that continuously express the E1 gene. This makes it possible to suppress replication of the virus in the target cells in the use as the vector and include a larger nucleic acid region as a payload (Non-Patent Literature 6).

However, it was not obvious how to shorten the backbone of an adenovirus vector, and there was difficulty in creating an adenovirus vector having a shorter backbone. For example, it is known that although the E3 gene itself is not essential for the replication of an adenovirus, this gene is adjacent to an essential structural protein on the genome, and deletion of too large a portion from the E3 gene cannot create a replicable adenovirus vector (Non-Patent Literature 7).

### Citation List

### Patent Literature

PTL 1: Japanese Patent Application Laid-Open No. 07-298877

### Non-Patent Literature

NPL 1: Wang et al., Annual Review of Biochemistry, 2016, Vol. 85: pp. 227-264
NPL 2: Ran et al., Cell, 2013, Vol. 154, pp. 1380-1389
NPL 3: Cong et al., Science, 2013, Vol. 339, pp. 819-823
NPL 4: Miyake et al., Proc. Natl. Acad. Sci., 1996, Vol. 93, pp. 1320-1324
NPL 5: Gulzman et al., In Eukaryotic viral vectors, 1992, pp. 187-192.
NPL 6: Bett et al., Proc. Natl. Acad. Sci., 1994, Vol. 91, pp. 8802-8806
NPL 7: Saito, Virus, 1997, Vol. 47, No. 2, pp. 231-238

### Summary of Invention

### Technical Problem

An object of the present invention is to provide a more excellent adenovirus vector used in, for example, gene therapy. In one aspect, an object of the present invention is to provide an adenovirus vector capable of delivering a longer nucleic acid region as a payload by modifying the backbone of the adenovirus vector. In another aspect, an object of the present invention is to provide a nucleic acid vector that expresses five or more Cas guide RNA, for use in, for example, genome editing by the CRISPR/Cas system. In another aspect, an object of the present invention is to provide an integration-type or all-in-one type nucleic acid vector (preferably an adenovirus vector or a lentivirus vector) containing Cas guide RNA expression units (preferably at least 3 units) and a Cas protein-coding gene, for use in, for example, genome editing by the CRISPR/Cas system. In another aspect, an object of the present invention is to provide a nucleic acid vector containing a Cas guide RNA expression unit, a Cas protein-coding gene, and a donor DNA sequence to be knocked in, for use in, for example, genome editing by the CRISPR/Cas system. In another aspect, an object of the present invention is to provide an adenovirus vector for simultaneously modifying many genes by combining backbone modifications of an adenovirus vector to express more kinds of Cas guide RNA.

### Solution to Problem

The present inventors conducted extensive studies to achieve the above objects and as a result have succeeded in shortening the backbone of a traditionally used adenovirus vector. Specifically, the present inventors have succeeded in increasing the deleted portion in the E3 region by 152 bases long from the traditionally used Ad5 vector.

The E3 region refers to the E3 gene and part of the L4 gene in the proximity thereof. The part of the L4 gene refers to bases from the start codon to one of the 125^{th} to 277^{th} bases in a pVIII precursor gene adjacent to the E3 gene group and belonging to the L4 gene group.

The adenovirus vector of the present invention having the novel backbone is useful as a vector for delivering a foreign gene having a longer coding sequence.

Also, the present inventors have succeeded in creating an adenovirus vector and a lentivirus vector that contain five or more, specifically eight or more guide RNA expression units and stably retain the guide RNA expression units to express the guide RNA in animal cells.

Also, the present inventors have succeeded in creating an adenovirus vector that contains a Cas protein-coding DNA fragment and three or more guide RNA expression units and is capable of simultaneously expressing these.

On the basis of these findings, the present invention has been completed.

That is, the present invention provides the following in one aspect.
[1] A recombinant adenovirus vector,
   wherein a region having a length of more than 2685 base pairs lacks from an E3 region.
[2] The recombinant adenovirus vector according to the above [1], wherein a region of 2837 base pairs lacks from the E3 region.
[3] The recombinant adenovirus vector according to the above [1], wherein the recombinant adenovirus vector is an Ad5 vector-derived recombinant adenovirus vector where 27982^{th} to 30818^{th} bases as positions in an Ad5 vector are deleted from the E3 region.
[4] A nucleic acid vector, which expresses five or more Cas guide RNA.
[5] The nucleic acid vector according to the above [4], wherein the nucleic acid vector expresses eight Cas guide RNA.
[6] The nucleic acid vector according to the above [5], wherein the nucleic acid vector is configured to introduce at least four double nicking to cause at least 4 genome cuttings.
[7] The nucleic acid vector according to the above [6], wherein the nucleic acid vector is designed to knock down a target sequence to be knocked down on a genome by introducing two double nicking into a region upstream and in proximity to the target sequence and further introducing two double nicking into a region downstream and in proximity to the target sequence.
[8] The nucleic acid vector according to any one of the above [4] to [7], wherein the nucleic acid vector is the recombinant adenovirus vector according to any one of the above [1] to [3].
[9] A nucleic acid vector, which is an integration-type Cas nucleic acid vector including a Cas protein-coding gene and a Cas guide RNA expression unit.
[10] The nucleic acid vector according to the above [9], wherein the nucleic acid vector includes three or more Cas guide RNA expression units.
[11] The nucleic acid vector according to the above [10], wherein the nucleic acid vector includes four Cas guide RNA expression units.
[12] The nucleic acid vector according to any one of the above [9] to [11], wherein at least one pair of the Cas guide RNA expression units is configured to cause double nicking.
[13] The nucleic acid vector according to any one of the above [9] to [12], wherein the nucleic acid vector is the vector according to any one of the above [1] to [8].
[14] A nucleic acid vector including:
   a Cas guide RNA expression unit;
   a Cas protein-coding gene; and
   a donor DNA sequence to be knocked in.
[15] The nucleic acid vector according to the above [14], wherein the nucleic acid vector includes two or more Cas guide RNA expression units, which are configured to introduce double nicking.
[16] The nucleic acid vector according to the above [14], wherein the nucleic acid vector includes eight Cas guide RNA expression units, which are configured to introduce four double nicking.
[17] The nucleic acid vector according to any one of the above [14] to [16], wherein the nucleic acid vector is the vector according to any one of the above [1] to [13].

### Advantageous Effects of Invention

Use of the adenovirus vector having the shortened novel backbone of the present invention makes it possible to transduce a longer DNA fragment than in traditional adenovirus vectors. For example, it is possible to use a gene encoding a longer protein in gene therapy, and it is possible to simultaneously express a larger number of guide expression units in the CRISPR/Cas system.

In therapy for genetic diseases and the like, knock-in is necessary that replaces an abnormal gene sequence with a normal gene sequence. A DNA fragment used for knock-in has normal base sequences used for homologous recombination at both sides (upstream and downstream) of an abnormal sequence. In general, these sequences are called a left-arm sequence (upstream) and a right-arm sequence (downstream). Use of the adenovirus vector having the shortened novel backbone of the present invention makes it possible to extend the lengths of these left-arm sequence and right-arm sequence in the DNA fragment used for knock-in. This results in an increased efficiency of homologous recombination, which is expected to increase efficiency of knock-in therapy.

Also, use of the nucleic acid vector of the present invention stably retaining eight or more Cas guide RNA expression units and simultaneously expressing them makes it possible to achieve not only a quantitative increase of the simultaneously expressed Cas guide RNA-coding DNA units but also a qualitative improvement of drastically increased usefulness in the usage of the double nicking system using this.

As described above, in order to delete a single DNA region from the genome with the double nicking system, four Cas guide RNA-coding DNA is necessary. Thus, only when expressing eight or more Cas guide RNA-coding DNA, it is possible to safely and highly efficiently perform gene disruption/insertion at two or more different sites on the genome at the same time. Use of such a single vector containing eight or more Cas guide RNA expression units has remarkable advantages in terms of vector transfection efficiency into the target cells, adjustment of the expression level, etc., as compared with, for example, a method using in combination two or more kinds of vectors each containing four Cas guide RNA expression units. This makes genome editing safer and highly efficient.

By performing gene disruption/insertion at two or more different sites on the genome by the double nicking method, for example, it is possible to disrupt two or more different genes at the same time. It has been revealed in many cancers that two or more different gene mutations are involved with canceration of cells. The ability to simultaneously edit two or more genes in genome editing therapy is understood to give remarkably beneficial outcomes in cancer therapy. By deleting two or more regions of a single gene, it is also possible to perform more reliable gene disruption.

Also, a virus is prone to mutation, and the base sequence of the genome of the virus infecting patients is different depending on the patients. In the case of a virus persistently infecting patients, it is known that the base sequence of the genome of a virus population in one patient is not the same but it is a population having various mutations. As a result, the virus having drug resistance selectively proliferates, causing a drug to lose its efficacy. Hepatitis B virus (HBV) is one example thereof, but this phenomenon is observed in not only HBV but also various pathogens such as other viruses, bacteria, and malaria, and is not limited to particular pathogens.

Therefore, when a vector targeting the virus genome expresses guide RNA multiply (expresses many different guide RNA), not only therapeutic effects are increased by simultaneously destroying many essential sites for the virus, but also the following effects are understood to be exhibited: (1) a single vector agent can be a broad-spectrum agent having effects on viruses of a wide range of patients having mutated viruses; (2) it reduces remaining viruses because it destroys each of the various viruses persistently infecting one patient; and (3) it simultaneously destroys many sites on the virus genome and thus does not give viruses any opportunity for passages enough to acquire resistances by mutations, resulting in not permitting emergence of resistant viruses. Regarding the effect of the above (1), see Example 4 in the present specification.

Similar effects are considered also in cancer. Specifically, not only therapeutic effects are increased by simultaneously disrupting many cell genes that facilitate proliferation of cancer cells, but also the following effects are understood to be exhibited: (1) a single vector agent can be a broad-spectrum agent having effects on cancers of a wide range of patients by having effects on different patients whose cells have undergone canceration due to different genome mutations; (2) it is likely to have an ability to disrupt each of various cancer cells even in one patient, such as cancer cells that have acquired a metastatic potential as a result of mutations of different genome sites and cancer cells that have increased drug resistance as a result of retarded proliferation; and (3) it simultaneously cuts and destroys many mutated sites on the genome of cancer cells and thus does not give cancer cells any opportunity for passages enough to acquire resistances by mutations, resulting in not permitting emergence of resistant cancer cells.

Also, the integration-type nucleic acid vector of the present invention (preferably an adenovirus vector or a lentivirus vector), which contains both a Cas protein-coding DNA fragment and Cas guide RNA expression units (preferably three or more), has remarkably beneficial effects in performing safe and highly efficient genome editing.

Traditionally, the RISPR/Cas system is delivered by co-infecting the target cells with a vector having a Cas protein-coding gene and a vector having Cas guide RNA expression units. Such a co-infection system is low in infection efficiency, and the success rate of genome editing in the target tissue is low. Meanwhile, the nucleic acid vector of the present invention (preferably an adenovirus vector or a lentivirus vector), which contains both a Cas protein-coding DNA fragment and Cas guide RNA expression units (preferably three or more) and is capable of simultaneously expressing these, can achieve highly efficient genome editing in the infected cells and avoid side effects that would otherwise occur when only either the Cas protein or the guide coding DNA is delivered.

Also, in the present invention, by combining the backbone modification of the adenovirus vector with eight or more Cas guide RNA expression units or combining a Cas protein-coding gene with Cas guide RNA expression units, it is possible to simultaneously express a larger number of Cas guide RNA-coding DNA to increase benefits of genome editing therapy.

### Brief Description of Drawing

FIG. 1 is an overview of genome editing by Cas9 nickase (nickCas9). One pair of nicks cause deletion in some cases.

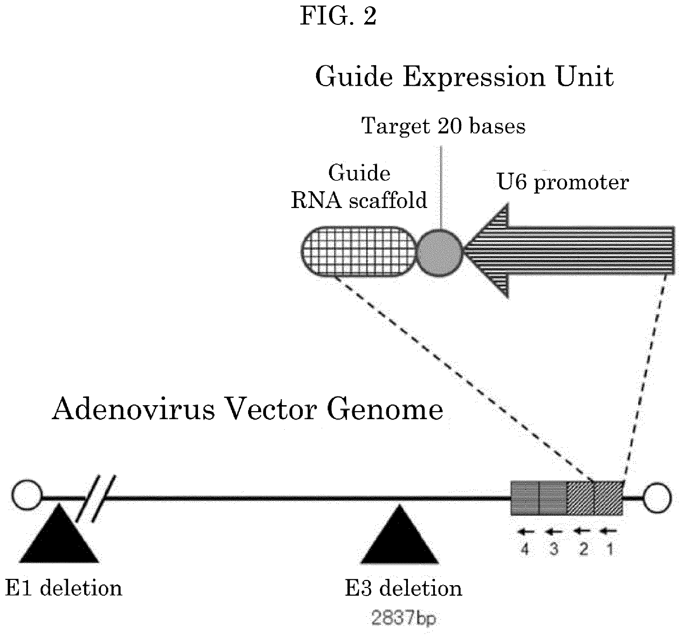

FIG. 2 is a view illustrating a structure of four guide RNA-coding DNA units (guide RNA expression units) and a structure of an adenovirus vector containing these.

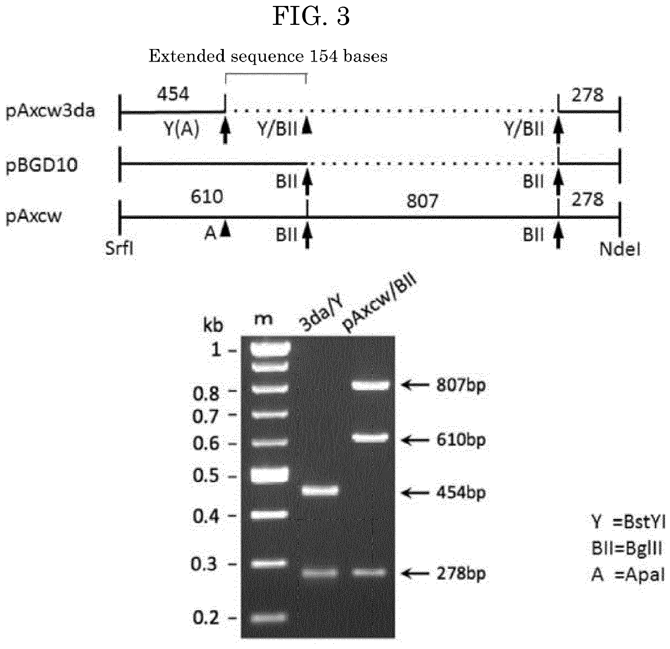

FIG. 3 is a view illustrating that deletion of the E3 region is confirmed through PCR.

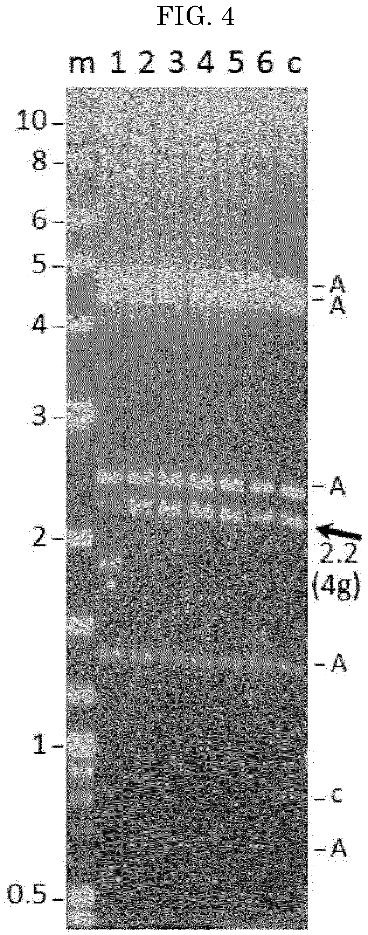

FIG. 4 is an agarose gel electrophoresis image illustrating that an adenovirus vector having four guide RNA expression units could be produced. BspEI was used to cut 293 cell DNA in which the adenovirus vectors produced by the full-length DNA introducing method were highly amplified. "m" denotes a marker, 1 to 6 denote each adenovirus vector clone, and the arrow denotes the position (2.2 kb) of a band of fragments each containing four guide units (4g). Clones 2 to 4 each retained four guide units without deletion. Most vectors of Clone 1 retained three units and deleted one unit (the band of which is denoted by *). "A" at the right-hand side of the image denotes bands derived from the adenovirus vector genome, and "c" denotes a band derived from a cosmid plasmid used for the production of the vector. Note that, in an experiment using another guide RNA set, all of the obtained six clones retained four guide units without deletion.

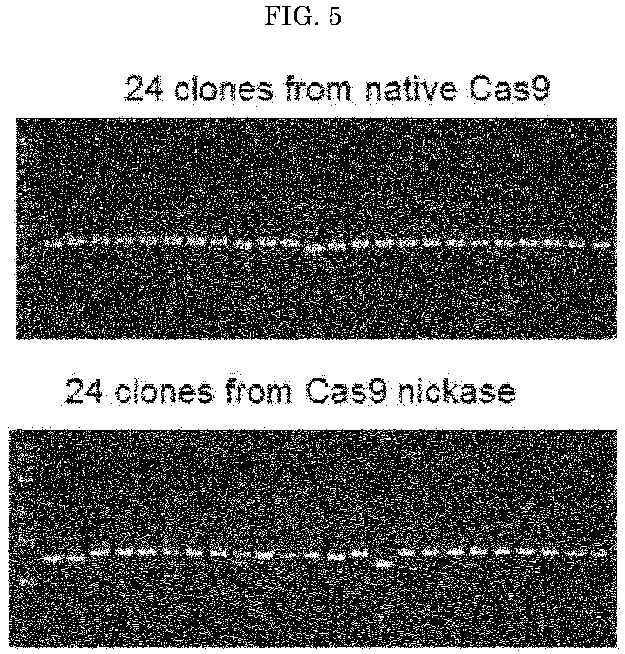

FIG. 5 is images illustrating genome editing results obtained using an adenovirus vector having four guide RNA expression units. Based on change (reduction) in the length of an amplified DNA fragment obtained by amplifying the target region through PCR, change in the target region occurring by genome editing was observed. The upper image illustrates results obtained in combination with an adenovirus vector expressing a native Cas9 protein (nuclease), and the lower image illustrates results obtained in combination with an adenovirus vector expressing Cas9 nickase. In FIG. 5, the leftmost denotes a marker, and the others denote adenovirus vector clones. In the case of the Cas9, apparently large deletion was found in one clone at the 12^{th} from the left. In the case of the Cas9 nickase, apparently large deletion was found in three clones at the 1^{st}, 2^{nd}, and 15^{th} from the left. These results were also confirmed in sequence analysis.

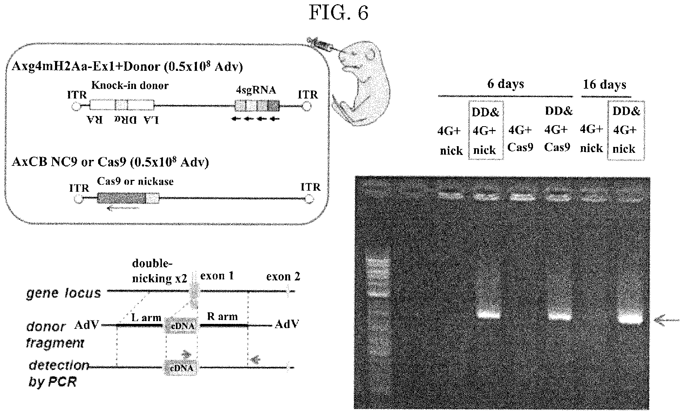

FIG. 6 is images illustrating genome editing results obtained using an adenovirus vector having four guide RNA expression units and a knock-in donor DNA (inserted). The knock-in donor sequence, which is contained on the same vector as the four guide RNA expression units (4sgRNA), is inserted onto the genome. The left-upper figure illustrates the structure of the co-infected vector. Through PCR using two primers on the donor DNA sequence indicated by the two arrows and the host cell base sequences outside it in the left-lower figure, bands detected only when accurate knock-in occurs were observed, as illustrated in the right-lower figure, six days later in the case of using Cas9 nickase (Lane 2 (6 days: DD&4G+nick)) and 16 days later (Lane 6 (16 days: DD&4G+nick)). The leftmost lane is a marker.

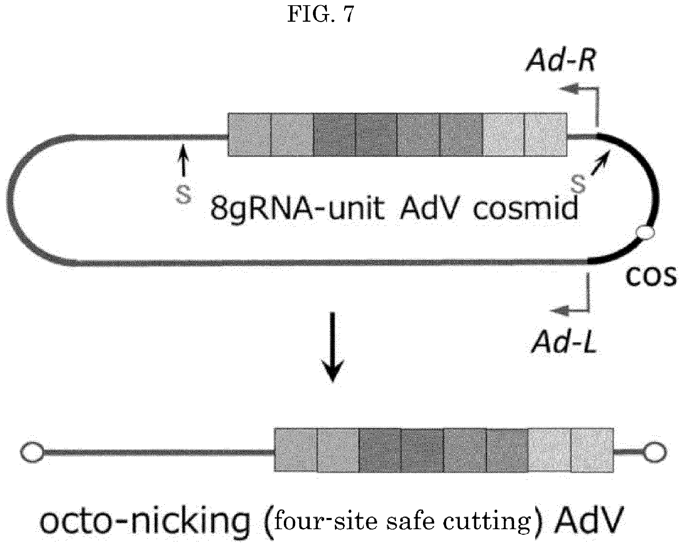

FIG. 7 is a view illustrating the structure of an adenovirus vector (the lower figure) having eight guide RNA expression units (8gRNA-unit) and the structure of a cosmid vector (the upper figure) used for creating this. In the figure, the squares denote the guide RNA expression units. Ad-L and Ad-R respectively denote the positions of the left-hand end and the right-hand end of the adenovirus genome of the vector. S denotes cutting positions by SspI for confirming that there is no deletion in the inserted eight guide expression units in proliferation of the cosmid plasmid in Escherichia coli. Note that, there are other SspI sites than those indicated here. COS denotes a sequence necessary for random in vitro packaging.

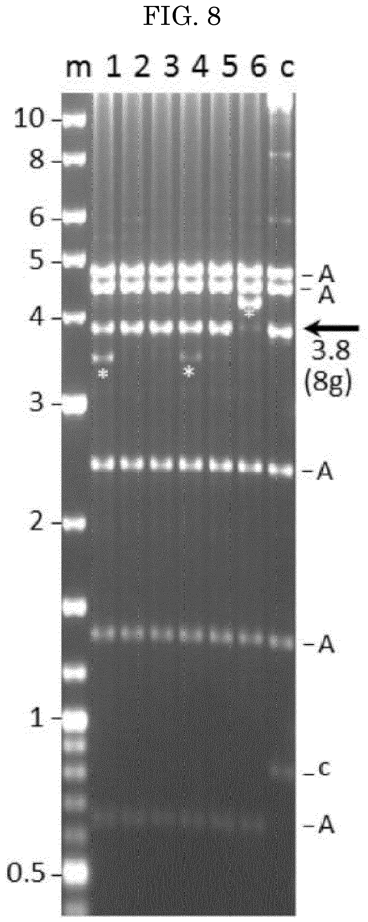

FIG. 8 is an agarose gel electrophoresis image illustrating that an adenovirus vector having eight guide RNA expression units could be produced. Similar to the description of FIG. 4, the vector-amplified cell DNA was cut by BspEI. "m" denotes a marker, 1 to 6 denote each adenovirus vector clone, and c denotes a control. The arrow denotes the position (3.8 kb) of a band of fragments each containing eight guide units (8g). "*" denotes a fragment containing units that are deleted or amplified through homologous recombination. Clones 2, 3, and 5 each retained eight guide units without deletion. Most vectors of Clones 1 and 4 retained eight guide units with almost no deletion, but some vectors deleted one unit and retained seven units, generating the bands indicated by "*". Interestingly, most vectors of Clone 6 retained nine guide units increased in the number of units through homologous recombination (as seen in the band indicated by "*"). "A" at the right-hand side of the image denotes bands derived from the adenovirus vector genome, and "c" denotes a band derived from a cosmid plasmid used for the production of the vector. Note that, in an experiment using another guide RNA set, all of the obtained six clones retained eight guide units without deletion.

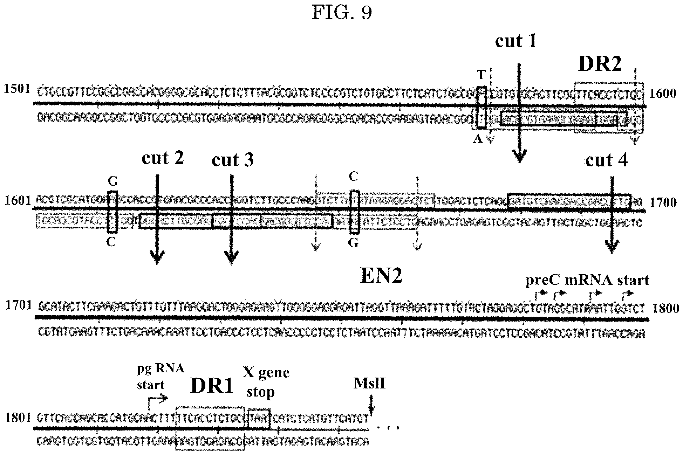

FIG. 9 is a view illustrating cutting positions of the X gene of HBV by an adenovirus vector simultaneously expressing eight guide RNA. The eight guide RNA expressed by the produced adenovirus vector are based on the genome sequence of a standard strain. However, HBV to target here is derived from a patient having a strain different from the standard strain, and difference of three bases is seen in the target sequence region. As a result, the four guide RNA cannot cut the target region due to this difference of bases, but the other four guide RNA sequences are homologous to the sequence derived from this patient and thus this vector can simultaneously cut the virus genome derived from this patient at four sites (cut 1 to cut 4 in the figure). In this way, it is considered that even if the HBV genome persistently infecting another patient has mutations at different positions, similarly, a multiple guide expression vector can simultaneously perform two or more cuttings. This target region is in the X gene assumed to be involved with the development of liver cancer by HBV. It not only disrupts this gene but also deletes and disrupts DR2 sequence, which is essential for virus genome replication, and a sequence downstream thereof.

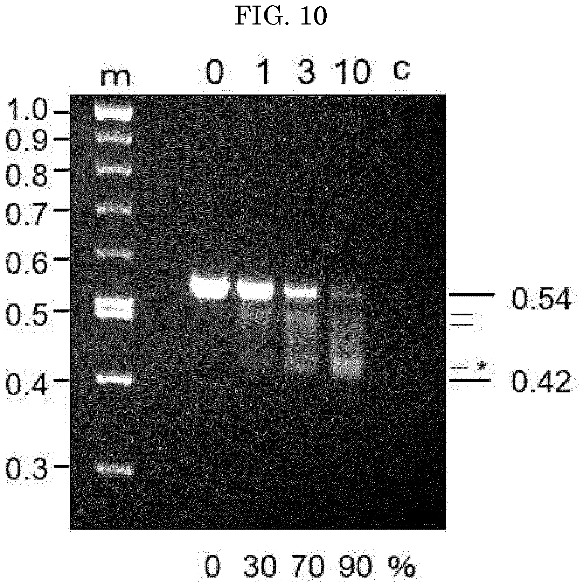

FIG. 10 is a view illustrating deletion of the target region in the X gene through co-infection of the adenovirus vector simultaneously expressing eight guide RNA and a Cas9-expressing adenovirus vector. The target region (from cut 1 to cut 4) in the X gene of HBV, which was incorporated into chromosomal DNA of HepG2 cells derived from liver cancer, was almost completely deleted at a virus load of moi 10. "m" denotes a marker, 0, 1, 3, and 10 denote moi, and "c" denotes a control. The numerical values (0, 30, 70, 90) under the figure denote a deletion disruption efficiency (%).

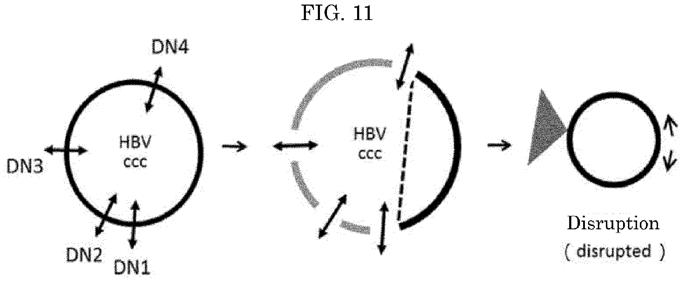

FIG. 11 is a view illustrating cutting positions of the HBV genome by the adenovirus vector simultaneously expressing eight guide RNA and simultaneously performing double-nicking cuttings at four sites. In the left-hand figure, DN1 to DN4 denote positions of double-nicking cuttings. A virus DNA fragment formed by cutting from the HBV genome during replication in HepG2 cells is bonded in the cells (as illustrated in the dashed line in the center figure) to be cyclized. The cyclized DNA (the right-hand figure) was detected as a linear PCR fragment by two PCR primers at the right-hand end. This figure illustrates the case where complete cuttings took place at DN1 to DN4. However, since the virus genome is replicating, there are also newly-formed uncut virus genomes (the left-hand figure).

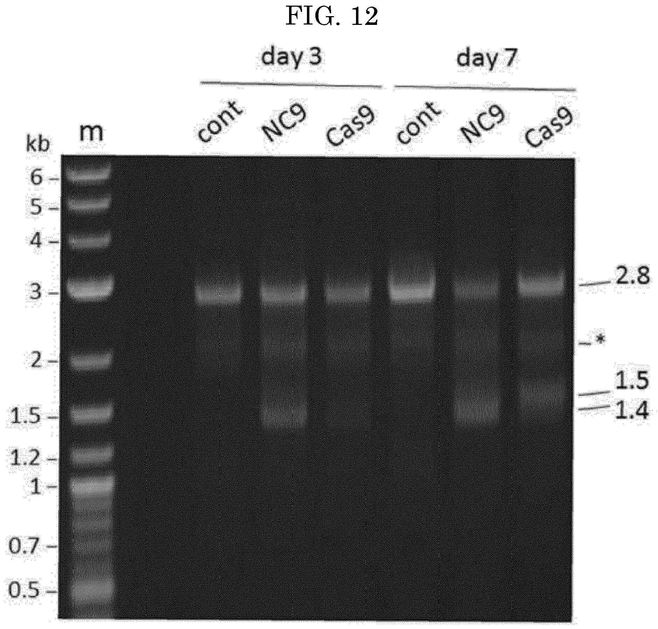

FIG. 12 is a gel electrophoresis image illustrating cuttings of the HBV genome by the adenovirus vector simultaneously expressing eight guide RNA and simultaneously performing double-nicking cuttings at four sites. This vector is co-infected to liver cancer-derived HepG2 cells together with a Cas9 nickase-expressing adenovirus vector to thereby perform double-nicking cuttings. Seven days after the co-infection (day 7), the replicating HBV genome amount (Lane NC9: the second from the right, the 2.8-kb band) was reduced to about 1/5 as compared with a control (Lane cont, the third from the right). Instead, a broad band of 1.4 kb formed when simultaneous cuttings take place at DN1 and DN4 was detected more densely. This result indicates that most of the HBV genomes are cut and disrupted and non-replicating HBV DNA is present more, suggesting that double-nicking cuttings were performed very efficiently. An experiment was also performed in parallel that used a Cas9-expressing vector instead of the Cas9 nickase-expressing vector. This case results in simultaneous cuttings at eight sites; i.e., four pairs of two proximal cutting sites. Interestingly, it was indicated that the double-nicking cutting inhibited the replication of the HBV genome more highly efficiently than cutting by Cas9 (from comparison between NC9, the second lane from the right, and Cas9, the rightmost lane, in terms of the density of the 2.8 kb band). In another experiment system using an adenovirus vector, it was also indicated that the double-nicking cutting disrupted the target more highly efficiently than Cas9 cutting.

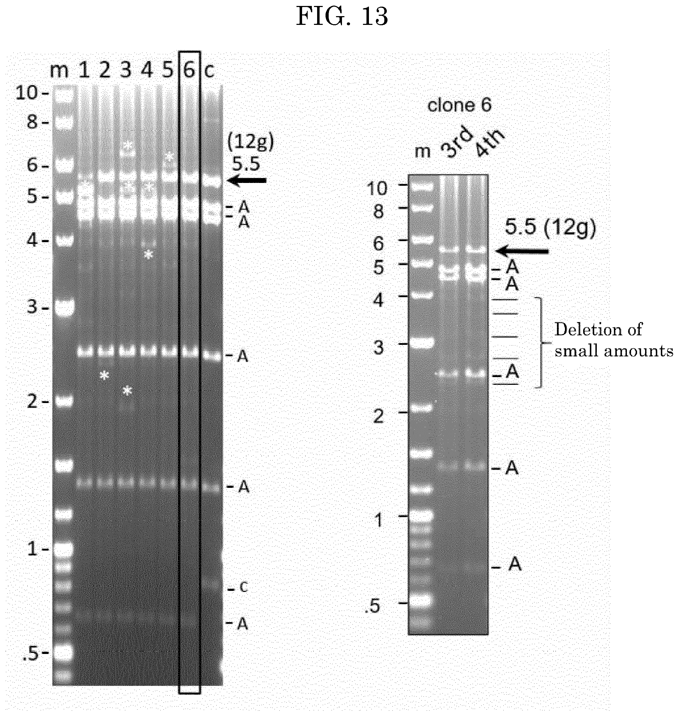

FIG. 13 is an agarose gel electrophoresis image illustrating that an adenovirus vector having 12 guide RNA expression units can be produced, where the produced vector involves no deletion even if amplified to such an amount that can perform a cell culture experiment. The left-hand figure illustrates results of six adenovirus vector clones, and the right-hand figure illustrates experiment results obtained by amplifying the vector of Clone 6 to study its stability. Similar to the description of FIG. 8, the vector-amplified cell DNA was cut by BspEI. "m" denotes a marker lane. In the left-hand figure, 1 to 6 denote each adenovirus vector clone in the 2nd-generation clones obtained by amplifying the 1st-generation clones, and c denotes a control. The arrow denotes the position (5.5 kb) of a band of fragments each containing 12 guide units (12g). "*" denotes a fragment containing units that are deleted or amplified through homologous recombination. The other clones than Clone 6 gave bands apparently indicating vectors with deletions indicated by "*", whereas Clone 6 (the framed lane) gave almost no band of vectors with deletions. "A" at the right-hand side of each image denotes bands derived from the adenovirus vector genome, and "c" denotes a band derived from a cosmid plasmid used for the production of the vector. The 3rd lane and the 4th lane in the right-hand figure indicate results of the 3rd-generation vector and results of the 4th-generation vector, respectively, where these vectors were obtained by amplifying the vector of Clone 6. The thin lateral lines at the right-hand side of the lanes in the figure indicate bands of small amount of vector fragments with deletions.

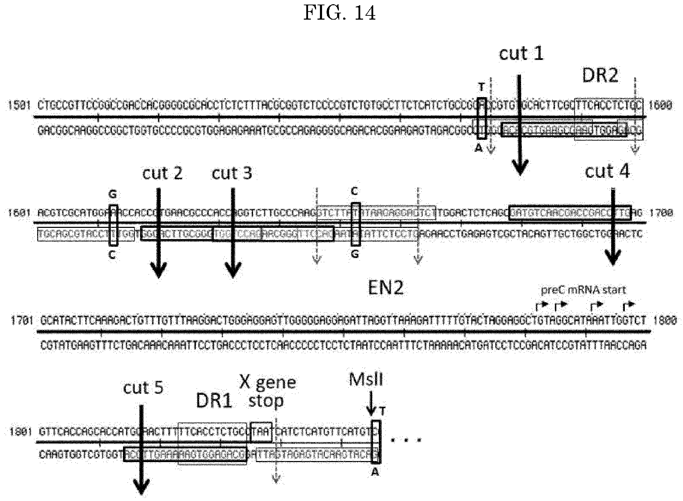

FIG. 14 is a view illustrating cutting positions of the X gene of HBV by an adenovirus vector simultaneously expressing 12 guide RNA. This vector further has four guide RNA expression units in addition to the eight guide RNA expression units illustrated in FIG. 8. Two of those four have the target sequences overlapping the sequence of the X gene near the stop codon of the X gene as illustrated in the lowermost sequence in the figure. One of the target sequences matches this gene sequence, and its cutting site is denoted by cut5. The other target sequence cannot be cut because one base at the 5' end (the last base in the figure) is different from the sequence of the X gene. The cutting position in the case where one base at the 5' end is not different is indicated with the vertical dotted arrow in the lowermost sequence.

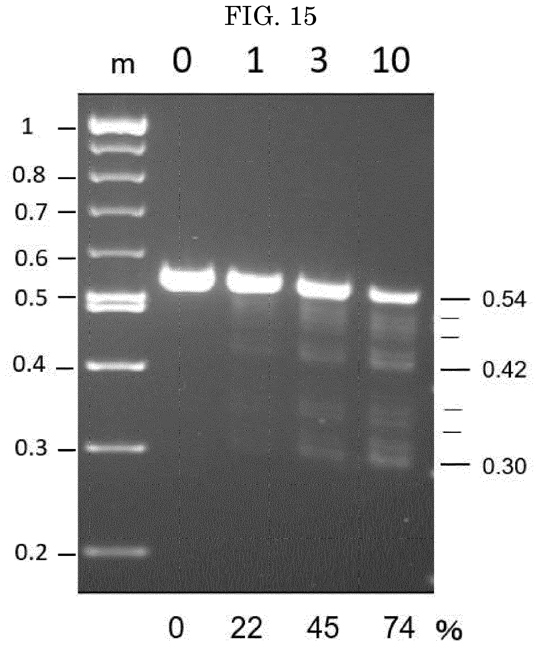

FIG. 15 is a view illustrating deletion of the target region in the X gene through co-infection of the adenovirus vector simultaneously expressing 12 guide RNA and a Cas9-expressing adenovirus vector. "m" denotes a marker and 0, 1, 3, and 10 denote moi. The thin lateral lines at the right-hand side of the lanes indicate bands occurring by combination of cut2 or cut3, which is slightly low in cutting efficiency, with cut1, cut4, or cut5. The numerical values (0, 22, 45, 74) under the figure denote a deletion disruption efficiency (%).

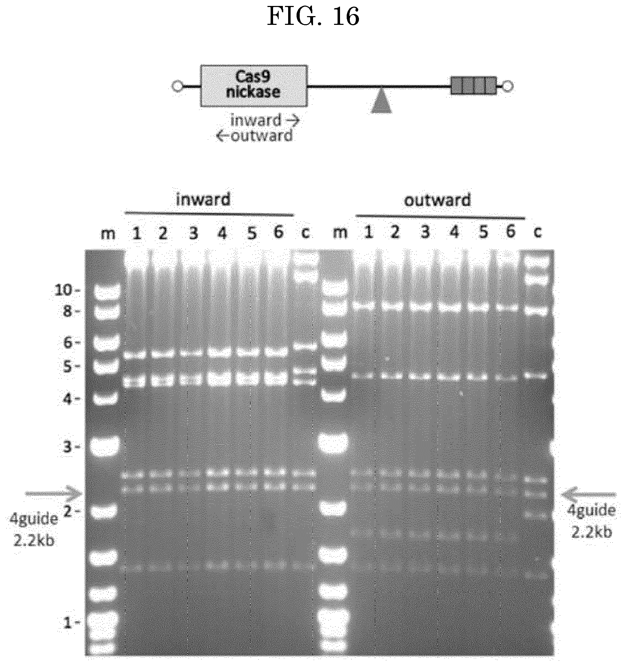

FIG. 16 is an agarose gel electrophoresis image illustrating that an adenovirus vector having a Cas9 nickase-coding gene and four guide RNA expression units could be produced. "m" denotes a marker, 1 to 6 denote each adenovirus vector clone, and "c" denotes a control, and "inward" and "outward" indicate that the transcription direction of the Cas9 nickase-coding gene is inward or outward. In either direction, all of the obtained six clones retained four units without deletion (as seen in the 2.2-kb bands indicated by the arrows). However, a band of three units due to deletion was slightly found in Clone 1 in the "inward" although it was almost invisible in the figure.

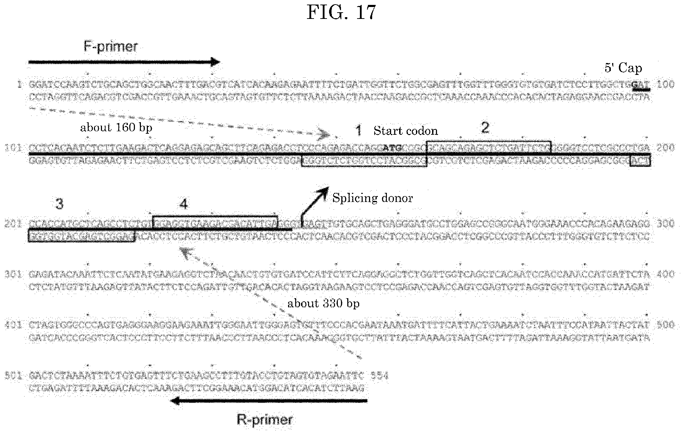

FIG. 17 is a view illustrating positions of four double-nicking guide RNA to cut mouse H2Aa gene exon 1 at two sites. Exon 1 contains a 5' cap site and a start codon, and the figure indicates positions of four guide RNA target sequences (1 to 4) to perform two pairs of double-nicking cuttings (DN cuttings) set downstream thereof.

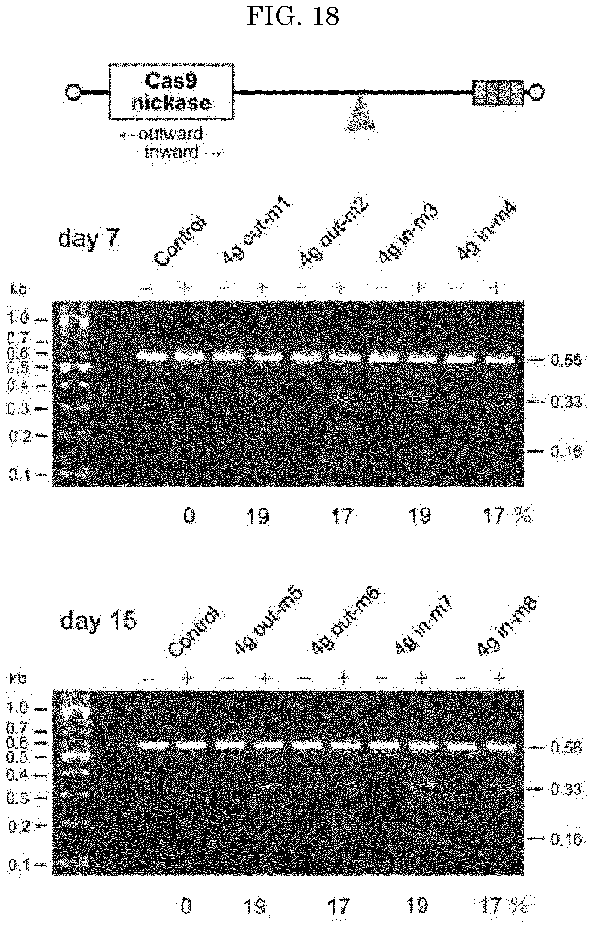

FIG. 18 is a view illustrating disruption (in vivo) of a mouse H2Aa gene using an integration-type adenovirus vector designed to perform double-nicking two-site cuttings with four guide RNA. Specifically, this figure illustrates a schematic view of the integration-type adenovirus vector, and agarose gel electrophoresis images illustrating the lengths of genome fragments of the target sites analyzed through T7E1 (T7 endonuclease I) assay seven days after administration of the vector (day 7) (Control, 4g out-m1, 4g out-m2, 4g in-m3, 4g in-m4) and 15 days thereafter (day 15) (Control, 4g out-m5, 4g out-m6, 4g in-m7, 4g in-m8). "+" means after T7E1 enzymatic reaction, and "-" means without addition of the enzyme. In addition to the 0.56-kb band indicating the unmodified genome fragments, the bands of about 0.33 kb and about 0.16 kb, which were not observed in the control (with no vector administered, the second lane from the left without taking the marker lanes into consideration (i.e., the lane of "+" of Control)), were observed on day 7 (4g out-m1, 4g out-m2, 4g in-m3, 4g in-m4) and day 15 (4g out-m5, 4g out-m6, 4g in-m7, 4g in-m8). These bands of about 0.33 kb and about 0.16 kb resulted from enzymatically removing the regions having undergone double nicking (DN regions). The numerical values (percent) under the lanes of "+" indicate a cutting removal efficiency of 0.56 kb DNA.

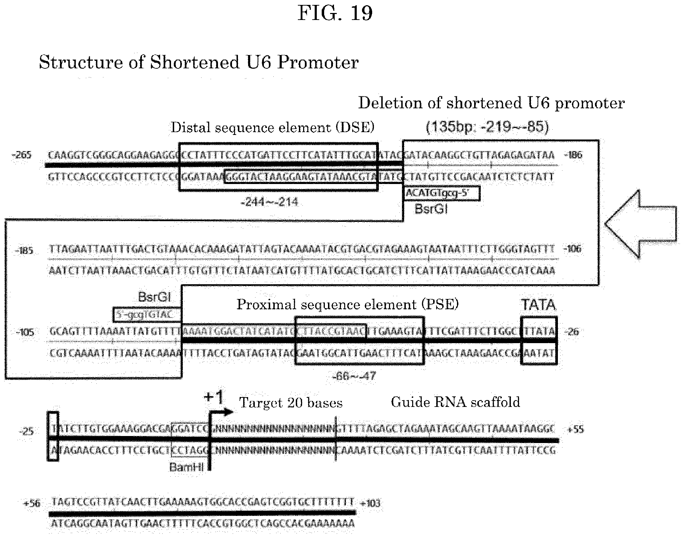

FIG. 19 is a view illustrating a structure of the guide RNA expression unit. The deleted portion in a U6 promoter (135 bp: -219 to -85) is in a frame indicated by the blank arrow.

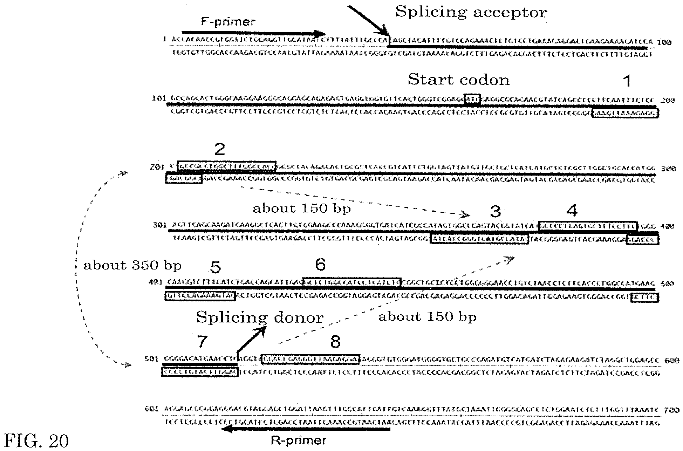

FIG. 20 is a view illustrating positions of eight double-nicking guide RNA to cut mouse NTCP gene exon 2 at four sites. Exon 2 contains a start codon, and the figure indicates positions of eight guide RNA target sequences (1 to 8) to perform four pairs of double-nicking cuttings (DN cuttings) set downstream thereof.

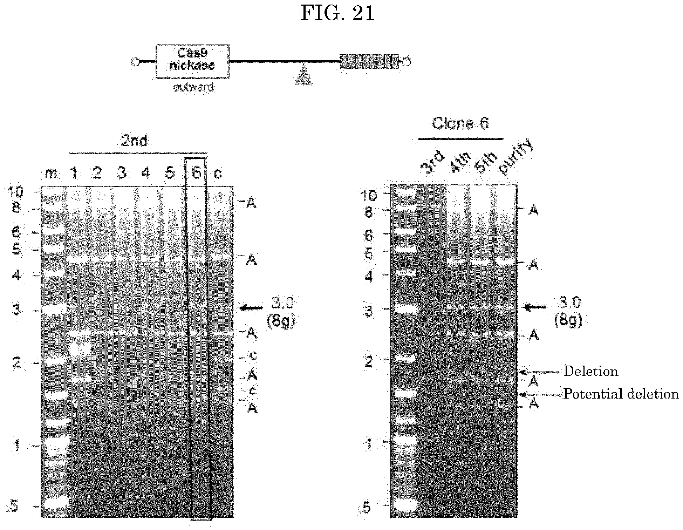

FIG. 21 is a view illustrating that an integration-type adenovirus vector having eight guide RNA expression units and Cas9 nickase expression units could be produced. Specifically, this figure illustrates a schematic view of the integration-type adenovirus vector and agarose gel electrophoresis images. In the left-lower figure of FIG. 21, the rightmost lane "c" denotes a cosmid used for the production of the adenovirus vector. In each of the lower figures of FIG. 21, "A" at the right-hand side of the gel indicates bands derived from the adenovirus vector and the cosmid and "c" indicates bands derived from the cosmid. The 3.0-kb band indicates that the vector has eight guide expression units. "2nd" in the left-hand gel and "3rd", "4th", and "5th" in the right-hand gel of FIG. 21 mean purifying each clone through passages twice, three times, four times, and five times, and "purify" means purified viruses of such an amount as to make an animal experiment possible. Most of the purified viruses gave the 3.0-kb band, revealing that they retained eight units.

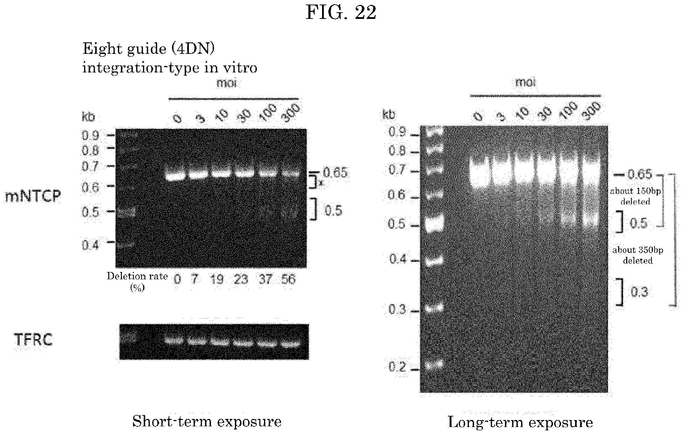

FIG. 22 is a view illustrating results (in vitro) of disruption of a mouse NTCP gene using an integration-type adenovirus vector designed to perform double-nicking four-site cuttings with eight guide RNA. The left-hand side is an agarose gel electrophoresis image exposed to light for a short time, and the right-hand side is an agarose gel electrophoresis image exposed to light for a long time. The fragment of 0.65 kbp indicates an unmodified genome fragment. The fragment of 0.5 kbp, in which about 150 bases are deleted from the fragment of 0.65 kbp, indicates deletion by simultaneous cutting of guide RNA1, 2 and guide RNA3, 4 or deletion by simultaneous cutting of guide RNA3, 4 and guide RNA7, 8. The fragment of 0.3 kbp, in which about 350 bases are deleted from the fragment of 0.65 kbp, indicates deletions between guides 1 and 2 and guides 7 and 8.

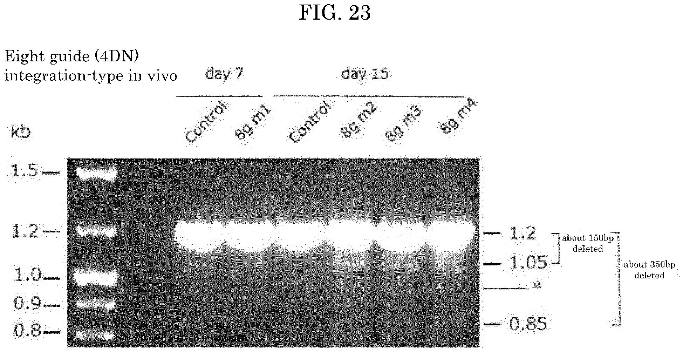

FIG. 23 is a view illustrating results (in vivo) of disruption of a mouse NTCP gene using an integration-type adenovirus vector designed to perform double-nicking four-site cuttings with eight guide RNA. The 1.05-kb and 0.85-kb bands were found in the 2nd, 3rd, and 4th mice on day 15.

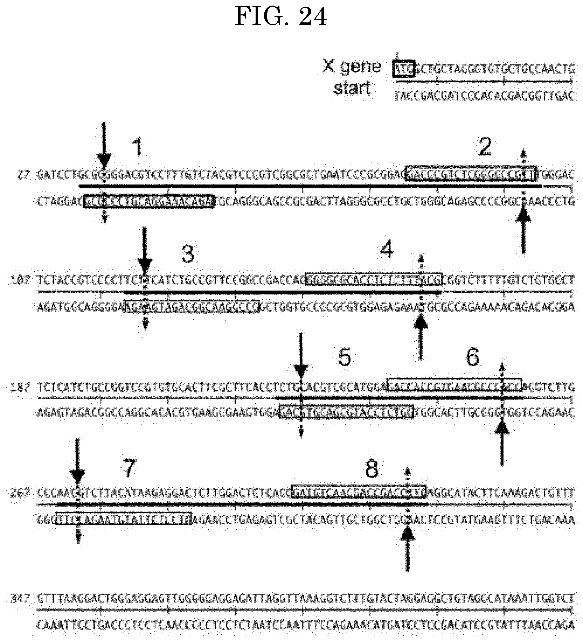

FIG. 24 is a view illustrating the target positions of eight guide RNA to perform four-site, double-nicking cuttings targeting the X gene. The eight guide RNA is denoted by numbers 1 to 8. Double-nicking sites are denoted by bold lateral lines. In FIG. 24, the solid-line arrows indicate positions of nicks by Cas9 nickase, and the dashed-line arrows indicate that cuttings of double strands take place at the indicated sites in the case of Cas9.

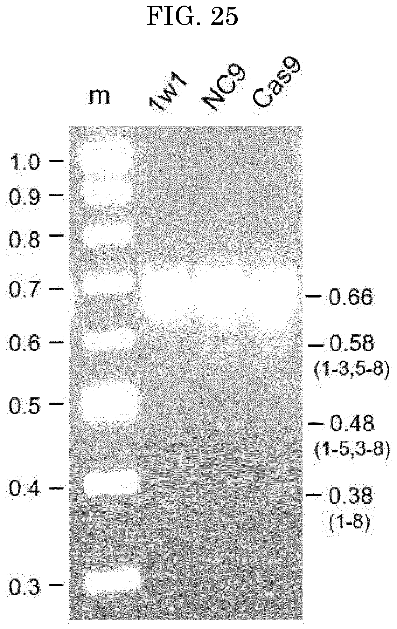

FIG. 25 is a view illustrating disruption of the X gene incorporated into a chromosome by a lentivirus vector expressing eight guide RNA and a Cas9- or Cas9 nickase-expressing adenovirus vector or a control adenovirus vector. The four-site, eight guide RNA target regions are amplified as 0.66 kb DNA fragments by forward and reverse primers sandwiching them. "m" denotes a marker, Cas9 indicates results obtained using a Cas9-expressing adenovirus vector, NC9 indicates results obtained using a Cas9 nickase-expressing adenovirus vector, and 1w1 indicates results obtained using a control adenovirus vector having no expression units.

### Description of Embodiments

Hereinafter, embodiments of the present invention will be described. The following descriptions are merely illustrative. The scope of the present invention should not be construed as being limited to these descriptions. The present invention can be practiced while appropriately modifying them in such a scope as not to impair the gist of the present invention.

### (Definitions)

In the present specification, when two or more numerical ranges are presented, a range is also meant that is formed by a combination of any of the lower limits and any of the upper limits in those numerical ranges.

In the present invention, "recombinant adenovirus vector" or "adenovirus vector" refers to an adenovirus containing, in the genome thereof, a base sequence other than the intrinsic base sequence of the adenovirus. The base sequence other than the intrinsic base sequence of the adenovirus is not particularly limited and may be a gene encoding a protein, a regulatory gene such as a promoter or a poly A sequence, or a base sequence having no function.

A foreign gene contained in the recombinant adenovirus vector of the present invention is, for example, a gene for therapy of human, a so-called reporter gene such as a GFP gene, or a recombinase. A position at which the foreign gene is to be inserted may be selected from an E1 region-deleted site, an E3 region-deleted site, and an insertion site upstream of an E4 gene, but the position is not limited thereto.

The origin of the recombinant adenovirus vector of the present invention is not particularly limited, but it is preferably an adenovirus derived from human. The adenovirus derived from human is more preferably type 2 or type 5 classified as type C.

A production method of the recombinant adenovirus vector is not particularly limited. It is preferable to use a method of inserting the intended gene into a cosmid vector containing the full-length adenovirus genome and then transfecting the resultant vector into 293 cells to obtain the recombinant adenovirus vector (the full-length DNA introducing method: Fukuda et al., Microbiol. Immunol. 2006, Vol. 50, pp. 463-454).

In the present invention, "genome editing" refers to a series of gene manipulations causing DNA to be inserted, substituted, or removed in the target DNA, for example, the genome of a cell, by use of one or more nucleases and/or nickases. A nuclease causes a specific double-strand cutting at a desired position on the genome and uses an endogenous mechanism of a cell to repair the caused cutting through homology directed repair (HDR) or non-homologous end-joining (NHEJ). A nickase causes a specific single-strand cutting at a desired position on the genome. In some embodiments, two nickases can be used to cause two single-strand cuttings in the strand facing the target DNA, to thereby form blunt or cohesive ends. Any suitable nuclease can be introduced to cause genome editing of the target DNA sequence. Examples thereof include, but are not limited to, CRISPR-associated protein (Cas) nucleases, Cpf1, zinc finger nucleases (ZFN), transcription activator-like effector nucleases (TALEN), mega nucleases, other endo- or exo-nucleases, variants thereof, fragments thereof, and combinations thereof.

In the present invention, "genome cutting" or "cutting of the genome" refers to cutting of both strands of a double-strand nucleic acid constituting the genome accompanied with the aforementioned genome editing. Cutting of the double-strand nucleic acid may result in formation of blunt ends through cutting at the base pair shared between the sense strand and the antisense strand or formation of cohesive ends through cutting at proximal different positions between the sense strand and the antisense strand.

In the present invention, "CRISPR/Cas system" refers to a system using CRISPR (clustered regularly interspaced short palindromic repeat) nucleic acid, which is an immune mechanism of prokaryotes, and a Cas protein (CRISPR-associated protein). "CRISPR/Cas system" is classified into class 1 and class 2, and "CRISPR/Cas system" of class 2 is further classified into type II, type V, and type VI (Yamano et al., Molecular Cell, 2017, Vol. 67, pp. 633-645).

A system classified as type II "CRISPR/Cas system" and using Cas9 as the Cas protein uses a Cas9 protein derived from Streptococcus pyogenes and a guide RNA (gRNA) composed of trans-activating crRNA (tracrRNA), which contains CRISPR RNA (crRNA) and trans-activating RNA having partial complementarity thereto and serving as a scaffold for binding to the Cas9 protein. The above crRNA and tracrRNA may be supplied as a fused single RNA molecule (single guide RNA) or may be supplied as separate RNA molecules (which may be referred to as "non-single guide RNA"). In one embodiment, "CRISPR/Cas9 system" is supplied to the target cells as a DNA fragment encoding a Cas9 protein, a DNA fragment encoding crRNA, and a DNA fragment expressing tracrRNA. In one embodiment, "CRISPR/Cas9 system" is supplied as a DNA fragment encoding a Cas9 protein and a DNA fragment encoding single guide RNA. In one embodiment, these DNA fragments are supplied to the target cells using a virus vector. These DNA fragments may be supplied to the target cells using a single virus vector or separate virus vectors. The DNA encoding tracrRNA is not particularly limited and may be appropriately selected from known ones. For example, a plasmid (pX260) available from Addgene and the like may be appropriately used. The specific DNA sequence of a part encoding tracrRNA in the plasmid is set forth in SEQ ID NO. 52.

In the present invention, "Cas protein" refers to a protein constituting "CRISPR/Cas system" and functioning as an effector molecule, and a derivative thereof. For example, "Cas protein" refers to a Cas9 protein, a Cpf1 protein, or a variant thereof having a DNA cutting activity. In one embodiment, "Cas protein" is a Cas nuclease having a DNA double-strand cutting activity. In one embodiment, "Cas protein" is a Cas nickase having an activity to cut only one strand of DNA double strands. In one embodiment, the Cas nickase may be a Cas9 (D10A) nickase having a mutation in a RuvC domain thereof (Jinek et al., 2012, Science, 2012, Vol. 337, no 6096, pp. 816-821).

In the present invention, "guide RNA expression unit" or "Cas guide RNA expression unit" refers to a DNA constituting unit for expressing guide RNA (which may also be referred to as "Cas guide RNA") used in the CRISPR/Cas system. In the case of using it in the CRISPR/Cas9 system, the guide RNA may be single guide RNA in which crRNA and tracrRNA are fused together or may be a non-single guide RNA in which crRNA and tracrRNA are separate. Embodiments of the "guide RNA expression unit" may include "single guide RNA expression unit" for expressing single guide RNA in which crRNA and tracrRNA are fused together, "crRNA expression unit" for expressing crRNA, and "tracrRNA expression unit" for expressing tracrRNA. The "guide RNA expression unit" may be a guide RNA expression unit formed only of "single guide RNA expression unit", may be a guide RNA expression unit formed of "crRNA expression unit" and "tracrRNA expression unit", or may be a guide RNA expression unit containing "single guide RNA expression unit", "crRNA expression unit", and "tracrRNA expression unit". The guide RNA expression unit contains not only DNA encoding the guide RNA but also, for example, expression regulatory regions that regulate its expression, such as a promoter. The promoter is not particularly limited and may be, for example, a U6 promoter, an H1 promoter, a tRNA promoter, or an adenovirus VA promoter. When two or more guide RNA expression units are incorporated into a vector, usually, these are adjacently arranged in tandem so as to be transcribed in the same direction.

In the present invention, that the Cas guide RNA expression unit is "configured to cause double nicking" refers to that at least two guide expression units are selected to introduce a single strand cutting; i.e., a "nick" in each of the sense strand and the antisense strand positioned in proximity on the genome, so that the double strand of the genome DNA are cut in the vicinity of the two nicks.

In the present invention, that two positions on DNA are "positioned in proximity" refers to that between the two positions are present, for example, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, and 50 base pairs, which are non-limitative.

In the present invention, "nucleic acid" is used as an equivalent to "nucleotide" or "polynucleotide" and refers to deoxyribonucleic acid (DNA), ribonucleic acid (RNA), and polymers thereof, in the form of a single strand, a double strand, or a multiple strand. The "nucleic acid" includes, but not limited to, single-strand, double-strand, or multiple-strand DNA or RNA, genome DNA, cDNA, and DNA-RNA hybrids. For example, the "nucleic acid" also includes polymers containing purine bases and/or pyrimidine bases, or other naturally-occurring, chemically-modified, biochemically-modified, not naturally-occurring, synthesized, or derivatized nucleotide bases. Unless otherwise specified, the nucleic acid having a specific sequence encompasses its conservatively modified variants (e.g., through substitution of degenerate codons), alleles, orthologs, single nucleotide polymorphisms (SNP), and complementary sequences.

In the present invention, the DNA sequence "encoding" specific RNA is a DNA nucleic acid sequence to be transcribed to RNA. A DNA polynucleotide may code for RNA to be translated to a protein (mRNA). Alternatively, the DNA polynucleotide may code for RNA not to be translated to a protein (e.g., tRNA, rRNA, or DNA-targeting RNA, which may also be referred to as "non-coding" RNA or "ncRNA).

In the present invention, "gene" is used as an equivalent to "a nucleotide sequence encoding a polypeptide" and refers to a DNA segment responsible for the production of a polypeptide chain. The DNA segment contains not only a region directly encoding a polypeptide but also regions before and after the coding region (leader and trailer) which are responsible for the regulations of its transcription and translation). The DNA segment may also contain an intervening sequence (intron) between the coding regions (exons).

In the present invention, "polypeptide", "peptide", and "protein" are used as equivalents to each other and refer to polymers of amino acid residues. The "polypeptide" includes naturally-occurring amino acid polymers, not naturally-occurring amino acid polymers, and polymers in which some amino acids are substituted with corresponding artificial chemical mimics.

In the present invention, "nucleic acid vector" refers to a molecule of nucleic acid used for delivering a foreign gene into the target cells. The nucleic acid vector is not particularly limited and may be a virus vector such as a plasmid, a DNA virus vector, or a RNA virus vector. The virus vector may be, for example, an adenovirus, a lentivirus, a baculovirus, a Sendai virus, a vaccinia virus, a herpes virus, or a variant thereof.

In the present invention, "double nicking" is a technique of forming nicks (single-strand cutting) in the sense strand and the antisense strand of double-stranded DNA at proximal positions, to thereby configure that the genome is cut at the intended site only when the nicks are formed at these two positions. A genome editing system including two or more guide RNA expression units constituted for using this technique may be referred to as "double-nicking system". The distance between the nicks is, for example, within 40 base pairs, and may be 1, 2, 3, 4, 5, 6, 7, 8, 9, 10, 15, 20, 25, 30, 35, and 40 base pairs, which are non-limitative.

By performing two "double nicking" at proximal positions, the genome cutting at the intended position can be made safer and more reliable. When two double nicking is introduced to proximal regions upstream of the target sequence to be knocked down from the genome and further two double nicking is introduced to proximal regions downstream of the target sequence, the target sequence can be knocked down very safely and reliably.

In the present invention, "integration-type Cas nucleic acid vector" or "all-in-one type Cas nucleic acid vector" refers to that the nucleic acid vector contains both the Cas guide RNA expression units and the Cas protein-coding gene. The "integration-type Cas nucleic acid vector" may be referred to simply as "integration-type nucleic acid vector" or "all-in-one type nucleic acid vector". Depending on the kind of the Cas protein, it may also be referred to as "integration-type Cas9 nucleic acid vector" or "all-in-one type Cas9 nucleic acid vector". Also, when the nucleic acid vector is, for example, an adenovirus vector, it may also be referred to as, for example, "integration-type Cas adenovirus vector".

The numbers of the Cas guide RNA expression units and the Cas protein-coding genes included in the "integration-type Cas nucleic acid vector" are not particularly limited. Typically, the "integration-type Cas nucleic acid vector" may include one copy of the Cas protein-coding gene and two or more, for example, 2, 3, 4, 5, or 6 Cas guide RNA expression units. A preferable embodiment is an embodiment where it includes four single guide RNA expression units and one Cas protein-coding gene, an embodiment where it includes two tracrRNA expression units, six crRNA expression units, and one Cas protein-coding gene, an embodiment where it includes four tracrRNA expression units, four crRNA expression units, and one Cas protein-coding gene, or an embodiment where it includes six tracrRNA expression units, two crRNA expression units, and one Cas protein-coding gene.

Also, the "integration-type Cas nucleic acid vector" of the present invention may further include a donor DNA sequence to be knocked in. The donor DNA sequence may be a sequence encoding a gene or may be a sequence not encoding a gene. The "integration-type Cas knock-in nucleic acid vector" can introduce the donor DNA sequence at a specific position on the genome with remarkably high efficiency. Typically, the integration-type Cas knock-in nucleic acid vector has a structure composed of one pair (two) of Cas guide RNA expression units for forming double nicking, one donor DNA sequence, and a gene encoding one Cas protein, but this structure is non-limitative.

In the present invention, "host cells" typically refer to cells into which a foreign DNA sequence has been transduced, and in particular, refer to cells suitable for the production of the nucleic acid vector of the present invention. Examples of the cells usable include microorganisms, yeast cells, insect cells, and mammalian cells. In particular, use of mammalian cells is preferable. Suitable examples of mammalian cells usable, but are not necessarily limited thereto, include HeLa cells, CHO cells, 293 cells, Vero cells, NIH3T3 cells, Huh-7 cells, BHK cells, PC 12 cells, COS cells, COS-7 cells, RAT1 cells, mouse L cells, Sf9 cells, human embryonic kidney (HEK) cells, and HepG2 cells.

In the present invention, "complementarity" refers to an ability of nucleic acid to form hydrogen bonds with a different nucleic acid sequence. The percentage of complementarity refers to a percentage of bases in a nucleic acid molecule that are able to form hydrogen bonds with a different nucleic acid sequence. "Completely complement" means that all of the consecutive residues of a nucleic acid sequence form hydrogen bonds with the same number of consecutive residues in a second nucleic acid sequence. As used in the present specification, that two nucleic acids are "substantially complement" means that the two nucleic acids are hybridized under stringent conditions.

In the present invention, "stringent conditions" refer to conditions under which nucleic acid having complementarity to the target sequence mainly hybridizes with that target sequence and does not substantially hybridize with a non-target sequence. In general, the longer a sequence, the higher the temperature at which the sequence specifically hybridizes with its target sequence.

In the present invention, "mutant" or "variant" refers to an organism, a strain, a gene, a polynucleotide, a polypeptide, or one form of its characteristics.

### (Adenovirus vector having shortened novel backbone)

The adenovirus vector having the shortened novel backbone of the present invention is a vector derived from adenovirus and is a recombinant adenovirus vector where a region having a length of more than 2685 base pairs lacks from an E3 region.

Such an adenovirus vector can be created based on an Ad5 vector by deleting in the E3 region of the Ad5, the 27982th base to the 30818th base adjacent to the cutting site by an ApaI restriction enzyme.

Deletion of this base sequence can be performed using a standard gene recombination technique that is usually used by persons skilled in the art.

The adenovirus vector having the shortened novel backbone can be amplified using, for example, cells that are usually used in combination with an Ad5 adenovirus vector (e.g., 293 cells).

### (Nucleic acid vector containing five or more guide RNA expression units)

The nucleic acid vector of the present invention containing five or more guide RNA expression units (expressing five or more Cas guide RNA) may be preferably a recombinant adenovirus vector or lentivirus vector.

A production method of the nucleic acid vector containing five or more guide RNA expression units is not particularly limited and may be appropriately selected depending on the intended purpose.

For example, a production method of an adenovirus vector containing five or more guide RNA expression units is not particularly limited. Preferably, it can be created by a method of inserting five or more guide RNA expression units each accompanied with gene expression regulatory sites into a cosmid vector containing part of adenovirus genome DNA and then transfecting the resultant vector into 293 cells to obtain the recombinant adenovirus vector (the full-length DNA introducing method: Fukuda et al., Microbiol. Immunol. 2006, Vol. 50, pp. 463-454).

A vector usable as the adenovirus vector is not particularly limited and may be various adenovirus-derived vectors.

In one aspect, it is desirable to use an Ad5 vector and a modified product thereof as the adenovirus vector. In one aspect, use of the adenovirus vector having the shortened backbone of the present invention can create an adenovirus vector containing a further larger number of guide RNA expression units.

Alternatively, the nucleic acid vector to be used may be other vectors such as a lentivirus vector. These vectors can be produced by a method that is usually performed by persons skilled in the art. In the case of a lentivirus vector, for example, it can be produced by a method described in Example 13 below.

In the nucleic acid vector of the present invention containing five or more guide RNA expression units, the number of the guide RNA expression units contained in the nucleic acid vector is not particularly limited as long as the number thereof is five or more but up to the number of the guide RNA expression units that can be contained in the nucleic acid vector, for example, may be 5, 6, 7, 8, 9, 10, 11, 12, 13, 14, 15, 16 or more. Specific examples of the embodiment of the "guide RNA expression unit" include an embodiment where it is "single guide RNA expression unit" and an embodiment where it is formed of "crRNA expression unit" and "tracrRNA expression unit". Examples of the embodiment where the "guide RNA expression unit" is formed of "crRNA expression unit" and "tracrRNA expression unit" include an embodiment where the "tracrRNA expression unit" is two and the "crRNA expression unit" is six, an embodiment where the "tracrRNA expression unit" is four and the "crRNA expression unit" is four, an embodiment where the "tracrRNA expression unit" is six and the "crRNA expression unit" is two, and an embodiment where the "tracrRNA expression unit" is four and the "crRNA expression unit" is eight.

The nucleic acid vector containing five or more guide RNA expression units may further contain a donor DNA sequence to be knocked in. The donor DNA sequence usable may be those in the "integration-type Cas nucleic acid vector" described above.

### (Nucleic acid vector containing Cas protein-coding gene and guide RNA expression unit)

The nucleic acid vector of the present invention containing a Cas protein-coding gene and a guide RNA expression unit can be created based on various nucleic acid vectors. In one aspect, the nucleic acid vector is preferably an adenovirus vector or a lentivirus vector. When it is created based on the adenovirus vector, it is preferable to use the above full-length DNA introducing method for the creation thereof, but this method is non-limitative. When using the full-length DNA introducing method using 293 cells, the culturing period of the 293 cells is preferably continued for 11, 12, 13, 14, 15, 16, 17, 18, 19, 20, 21, 22, 23, 24 days or longer. When the nucleic acid vector of the present invention is created based on the lentivirus vector, it can be created in the same manner as in the method described in Example 13 described below.

The positional relationship between the Cas protein-coding gene and the guide RNA expression unit may be appropriately selected. For example, the guide RNA expression unit may be positioned upstream of the Cas protein-coding gene, or the guide RNA expression unit may be positioned downstream of the Cas protein-coding gene.

The Cas protein is not particularly limited and may be various Cas proteins. Preferably, Cas9 nuclease or Cas9 nickase may be used.

The number of the guide RNA expression units contained in the nucleic acid vector of the present invention containing the Cas protein-coding DNA fragment and the guide RNA expression unit is not particularly limited as long as it is the number of the guide RNA expression units that can be contained in the nucleic acid vector. Preferably, three, four, five, six, seven, eight or more guide RNA expression units can be incorporated. Specific examples of the embodiment of the "guide RNA expression unit" include an embodiment where it is "single guide RNA expression unit" and an embodiment where it is formed of "crRNA expression unit" and "tracrRNA expression unit". Examples of the embodiment where the "guide RNA expression unit" is formed of "crRNA expression unit" and "tracrRNA expression unit" include an embodiment where the "tracrRNA expression unit" is two and the "crRNA expression unit" is six, an embodiment where the "tracrRNA expression unit" is four and the "crRNA expression unit" is four, an embodiment where the "tracrRNA expression unit" is six and the "crRNA expression unit" is two, an embodiment where the "tracrRNA expression unit" is four and the "crRNA expression unit" is eight, and an embodiment where the "tracrRNA expression unit" is two and the "crRNA expression unit" is two. The nucleic acid vector of the present invention containing the Cas protein-coding DNA fragment and the guide RNA expression unit may be preferably a recombinant adenovirus vector or lentivirus vector.

The adenovirus vector of the present invention containing the Cas protein-coding DNA fragment and the guide RNA expression unit can be produced based on various adenovirus vectors. For example, use of the adenovirus vector having the shortened backbone structure of the present invention can further increase the number of guide RNA expression units to be contained.

The nucleic acid vector of the present invention containing the Cas protein-coding DNA fragment and the guide RNA expression unit can be produced and amplified in various host cells. The nucleic acid vector of the present invention containing the Cas protein-coding DNA fragment and the guide RNA expression unit can exhibit genome editing activity of the Cas protein in the host cells as well. Thus, it is preferable to culture under such culturing conditions under which this activity is less likely to occur. It is also desirable to culture under such culturing conditions under which expression of the Cas protein can be suppressed. In one aspect, by introducing to the culture cells, a vector expressing an siRNA to suppress the expression of the Cas protein, it is possible to suppress the expression of the Cas protein in the production step of the nucleic acid vector of the present invention. Preferably, the siRNA is used in two or more different regions of the Cas protein-coding gene, for example, in 2, 3, 4, 5, 6 or more regions thereof.

The nucleic acid vector or adenovirus vector of the present invention not only can be used in genome editing experiments by applying an effective amount thereof to culture cells, but also can be applied to, for example, genome editing experiments or treatments of diseases by administering an effective amount thereof to animals excluding human and treatments of diseases through genome editing by administering an effective amount thereof to human.

### (Treatment method)

In the present invention, examples of "administering" include oral administration to a subject, local contact, administration as a suppository, and intravenous, intraperitoneal, intramuscular, intralesional, intrathecal, intranasal, or subcutaneous administration. The administration is by any route including parenteral and transmucosal routes (e.g., buccal, sublingual, palatal, gingival, nasal, vaginal, rectal, or transdermal route). Examples of the parenteral administration include intravenous, intramuscular, intraarteriole, intradermal, subcutaneous, intraperitoneal, intraventricular, and intracranial administrations. Other manners of delivery include, but are not limited to, use of liposome preparations, intravenous injection, and transdermal patch.

In the present invention, "effective amount" or "sufficient amount" refers to an amount of an active substance enough to cause beneficial or desirable outcomes (e.g., Cas nuclease or guide RNA). A therapeutically effective amount can be varied depending on a subject under treatment and one or more of disease condition of the subject, the body weight and age of the subject, severity of the disease condition, a manner of administration, etc. The therapeutically effective amount can be determined by persons skilled in the art. A specific amount can be varied depending on one or more of the selected specific active substance, target cell type, location of the target cell in a subject, a regimen to follow, whether the selected specific active substance is administered in combination with other active substances, the timing of administration, and a physical delivery system for delivering it.

In the present invention, "pharmacologically acceptable carrier" refers to a substance that aids administration of an active substance (e.g., Cas nuclease or guide RNA) to cells, organisms, or subjects. Examples of the pharmacologically acceptable carrier include, but are not limited to, water, NaCl, physiological saline, Linger's lactate solution, the defined sucrose, the defined glucose, a binding agent, a volume expander, a disintegrating agent, a lubricant, a coating agent, a sweetening agent, a perfume, and a colorant.

### Examples

The present invention will be described below in more detail by way of Examples, which should not be construed as limiting the present invention thereto.

### [Example 1]

### Production of adenovirus vector having shortened novel backbone

A commercially available E1/E3 lacking-type Ad5 vector was used to produce a recombinant adenovirus vector lacking the 27982th base to the 30818th base as positions in an Ad5 vector in the E3 region.

A production method thereof is as follows. A SpeI (27082)-NdeI (31089) fragment was cloned into a plasmid from the adenovirus genome of commercially available pAxcwit2. This plasmid was subjected to dual cutting with ApaI (27984) and BglII (30819) and treated with a Klenow polymerase to blunt both ends, to thereby obtain a plasmid lacking 2837 base pairs from the E3 region. The following three fragments are simultaneously bonded to obtain plasmid pAxcw3dait2 including the above E3 region lacking and retaining the rest adenovirus DNA: i.e., a fragment obtained by cutting pAxcwit2 with SpeI and NdeI and lacking the cut region, and a NdeI-PmeI (13258) fragment and a PmeI-SpeI fragment each containing a plasmid portion from pAxcwit2. This plasmid was cut with NdeI to prepare a fragment including an E3-lacking region. After that, pAxc4wit2 (Suzuki et al., Gene Ther., 2015, Vol. 22, pp. 421-429) having a SwaI cloning site in an E4 region was similarly cut with NdeI to replace the E3 region, to thereby obtain plasmid pAxc3da4w for creating an adenovirus vector having the above lack in the E3 region and the SwaI cloning site in the E4.

Deletion of the E3 region was confirmed through comparison by PCR-amplifying a region of the commercially available pAxcwit2 corresponding to the region of the above pAxc3da4w having a sequence of the same region as the E3 region-lacking adenovirus vector (FIG. 3). Also, the sequence of this lacked portion was confirmed through sequencing.

The adenovirus vector was confirmed to be replicable in 293 cells.

A foreign gene expression unit was incorporated into this plasmid, and the both ends of the adenovirus genome were cut with PacI, followed by transfection into 293 cells, so that an adenovirus vector expressing the foreign gene could be obtained. One example thereof is illustrated in FIG. 9; i.e., an adenovirus vector having a Cas9 nickase expression unit in the E1 region and four guide RNA expression units (single guide RNA) at insertion sites upstream of the E4 gene.

### [Example 2]

### Genome editing by adenovirus vector simultaneously expressing two or more guide RNA

The adenovirus genome is double-stranded linear DNA of about 36 kbp and has a unique structure in which 55 k proteins obtained by cutting E2B gene products are covalently bonded to both ends of the DNA strand. In order to create an adenovirus vector simultaneously expressing two or more guide RNA, properties of a lambda phage to incorporate only the 38 kb to 52 kb DNA fragment having a cos site were used to create a cosmid plasmid in which fragments of the adenovirus genome and four guide RNA expression units were connected in tandem. The full-length vector genome was cut out from the plasmid to transfect 293 cells (the full-length DNA introducing method) to create an adenovirus vector containing four guide RNA expression units (single guide RNA).

The guide RNA targets exon 1 of a mouse H2-Aα gene, and the multiple guide RNA expression units contained a first double-nicking guide coding DNA sequence formed of 5'-GCGGCATCCTGGTCTCTGGG-3' (SEQ ID NO. 1) and 5'-GCAGCAGAGCTCTGATTCTG-3' (SEQ ID NO. 2) and a second double-nicking guide coding DNA sequence formed of 5'-GAGGCTGAGATGGTGGTCA-3' (SEQ ID NO. 3) and 5'-GGAGGTGAAGACGACATTGA-3' (SEQ ID NO. 4) (FIG. 2).

Each of the guide RNA expression units contained a U6 promoter (about 261 base pairs) and a scaffold portion for guide RNA (a sequence other than the target 20 base pairs) (about 83 base pairs) in addition to the above guide coding DNA sequence.

When an adenovirus vector (AdV) was created using the above-created cosmid having multiple guide RNA expression units, it was possible to create the adenovirus vector without deletion of the guide RNA expression units (FIG. 4).

This AdV could be scaled-up to such an amount as to perform animal experiments. Utilizing the fact that most of the AdV infect the liver when the AdV is administered to mice from their veins, the AdV expressing four guide RNA was vaccinated to mice together with the AdV expressing Cas9 or Cas9 nickase, to analyze the genome editing efficiency of the target gene in the liver.

As a result, gene disruption was observed in the liver with high efficiency, and deletion of a larger region was observed in the combination with the Cas9 nickase expression vector as compared with the combination with the Cas9 expression vector, suggesting that use of the Cas9 nickase expression vector achieved more complete gene disruption (FIG. 5). Also, it was found that by further containing donor DNA in the multiple guide RNA expression AdV, it was possible to achieve gene knock-in of the donor DNA to the target site (FIG. 6).

These results indicate that use of an adenovirus vector expressing many guide RNA can perform highly safe in vivo cancer gene disruption experiments and genome editing therapy experiments of genetic diseases.

### [Example 3]

### Creation of adenovirus vector simultaneously expressing eight guide RNA

The full-length DNA introducing method was used to attempt to create an adenovirus vector containing eight guide RNA expression units targeting the DR2 region in the X gene of HBV (single guide RNA) (see FIG. 7). The guide coding DNA sequences used were set forth in SEQ ID NOs. 5 to 12.
SEQ ID NO. 5 GAAGCGAAGTGCACACGGTC
SEQ ID NO. 6 GAGGTGAAGCGAAGTGCACA
SEQ ID NO. 7 GGTTTCCATGCGACGTGCAG
SEQ ID NO. 8 GGCAGATGAGAAGGCACAGA
SEQ ID NO. 9 GAAGCGAAGTGCACACGGTC
SEQ ID NO. 10 GAGGTGAAGCGAAGTGCACA
SEQ ID NO. 11 GGTTTCCATGCGACGTGCAG
SEQ ID NO. 12 GACCTGGTGGGCGTTCACGG

It is known that DNA introduced into cells causes homologous recombination, and knock-in is widely performed using this phenomenon. In the COS-TPC method which has traditionally been performed in the adenovirus genome, an expression unit-containing cosmid and a cut parental virus DNA-terminal protein complex (DNA-TPC) are co-transduced into 293 cells at the final stage, and homologous recombination occurring between the common sequences of the cosmid and the parental virus DNA-terminal protein complex is utilized to recover, as an adenovirus vector, a virus DNA-terminal protein complex containing the intended DNA fragment.

In view thereof, when creating an adenovirus vector containing eight guide RNA expression units, it was expected that part of these expression units would lack due to homologous recombination because they contained common DNA sequences.

Nonetheless, the results of the experiment surprisingly indicated that an adenovirus vector containing eight guide RNA expression units could be created without lacking of any expression unit (FIG. 8).

A method commonly performed for creating an adenovirus vector includes transfecting 293 cells with the virus genome incorporating foreign DNA, and preparing the resultant viruses as a pool without individually separating them. As seen in FIG. 8, however, occurrence of a virus lacking some units is unavoidable, and thus there is a need to obtain a virus as an individual clone. Specifically, the transfected 293 cells are scraped from the culture dish and diluted on the following day, and mixed with a large number of non-transfected 293 cells and seeded again on a 96-well plate. Viruses were separated from only the plates where the number of the 293 cells killed by proliferating viruses was 1/3 or less of the 96-well plate.

This first step of separating a virus stock gave a virus stock with the units deleted and a stock retaining the eight units as illustrated in FIG. 8. When 293 cells were infected with only the virus stock retaining the eight units at a greater step, infecting the 293 cells with the virus stock in an amount three times the usual amount could provide a high-concentration virus stock without the deletion of the units. By repeating virus amplification under the above infection conditions, it was possible to obtain a deletion-free virus vector in an enough amount for animal experiments.

### [Example 4]

Efficient disruption of HBV-X gene on the chromosome by adenovirus vector

### simultaneously expressing eight guide RNA

A HBV-X gene is considered to involve the onset of liver cancer by this virus, and the X gene DNA is incorporated into liver cancer cells. As a model case of liver cancer therapy, HepG2 cell line Gx11 (Shintani, Saito, Koike et al., J. Gen. Virol. 1999, Vol. 80, pp. 3257-3265) incorporating the X gene on the chromosome thereof was co-infected with the adenovirus vector simultaneously expressing eight guide RNA created in Example 3 (eight guide expression vector) and the Cas9-expressing adenovirus vector in an attempt to disrupt the X gene.

The eight guide RNA expressed by the eight guide expression vector is created based on the base sequence of the genome of a standard-strain virus (the base sequence of FIG. 9). Due to the difference in the virus genome sequence between patients, however, the sequence of the virus incorporated into the Gx11 cell chromosome is different by three bases from the base sequence of the genome of the standard-strain virus in the base sequence of the target region in this region (in FIG. 9, A→T at the first row from the top, and A→G and T→C at the second row; these bases are framed, and the bases in the Gx11 cell line are given outside of each frame). The target sequences of 20 bases each of these eight guide RNA are overlapped with each other, and four guide RNA out of the eight guide RNA cannot be cut due to the differences of these bases (dashed-line arrows). However, the base sequence of the genome of the standard-strain virus matches the virus genome base sequence on the Gx11 cell chromosome in the base sequences of the rest four guide RNA, and these regions can be cut at respective target sites (the cutting positions are denoted by cut1 to cut4). Cuttings at all of the positions of cut1 to cut4 results in deletion of about 120 bases between cut1 to cut4. The fourth row from the top in FIG. 9 indicates the stop codon TAA of the X gene and the X gene-coding region upstream thereof in bold. Deletion in this bold region allows the X gene to lose the functions thereof. MslI at the rightmost of the fourth row indicates a position of the terminus of the virus DNA incorporated into Gx11 cells.

An experiment was performed in which the eight guide expression vector and the Cas9-expressing vector were co-infected in different amounts. As a result of PCR performed using PCR primers containing this region, at a virus load of 3 (expressed as moi), reduction in the band of the intrinsic size (0.53 kb) was clearly observed as compared with the case of non-infection (a moi of 0). At a moi of 10, this band disappeared almost completely, and became a 0.42-kb band observable as a result of deletion of 110 bases (see FIG. 10). This result indicates that disruption of the X gene on the chromosome by the eight guide expression vector and the Cas9-expressing vector was performed with high efficiency. The sequences of the primers used for PCR are set forth in SEQ ID NOs. 13 and 14.
Forward primer:
   SEQ ID NO. 13 ACTGGATCCTGCGCGGGACGTCCTTTGTC
Reverse primer:
   SEQ ID NO. 14 GAAAAAGATCTCAGTGGTATTTGTGAGCCAGG

### [Example 5]

### Disruption by cutting of replicating HBV genome by eight guide expression vector designed to perform four-site, double-nicking cutting

A HBV takes a form of the circular double-stranded DNA genome (ccc) during the process of replication. An eight guide RNA (single guide RNA) expression vector was created which performs double-nicking cutting at four sites of the replicating and amplifying HBV ccc genome. The presumed cutting sites are illustrated in the left-hand figure of FIG. 11. "DN" refers to double nicking. The guide coding DNA sequences used were SEQ ID NOs. 15 to 22.
DN1 site:
   SEQ ID NO. 15 GACCTGGTGGGCGTTCACGG
   SEQ ID NO. 16 GTCTTATATAAGAGGACTCT
DN2 site:
   SEQ ID NO. 17 GACATGAACATGAGATGATT
   SEQ ID NO. 18 GCTGTGCCTTGGGTGGCTTT
DN3 site:
   SEQ ID NO. 19 GATTGAGACCTTCGTCTGCG
   SEQ ID NO. 20 GTCGCAGAAGATCTCAATCT
DN4 site:
   SEQ ID NO. 21 GATATGGGTGAGGCAGTAGT
   SEQ ID NO. 22 GTCAATCTTCTCGAGGACTG

When the replicating HBV ccc genome undergoes double-nicking cutting at four sites, four DNA fragments are formed in the case of complete cutting. Each of the fragments is cyclized when both of the ends thereof are bonded to each other by the repair mechanism in cells (in the center figure of FIG. 11, the dashed line indicates binding sites of the largest fragment). Each cyclic DNA has most of the HBV genome deleted, and cannot replicate. The largest cyclic DNA (FIG. 11, right-hand side) was detected through PCR. In this case, the replicating full-length cyclic DNA genome (FIG. 11, left-hand side) that just forms by the replicative HBV genome-producing adenovirus vector and undergoes no cutting is also detected. The sequences of the PCR primers were SEQ ID NOs. 23 and 24.
Forward primer:
   SEQ ID NO. 23 TTAACTTCATGGGATATGTAATTGG
Reverse primer:
   SEQ ID NO. 24 ACTCAAGATGTTGTACAGACTTGGC

In order to obtain the HBV replicating genome, HepG2 cells were infected at a moi of 3 with the replicative HBV genome-producing adenovirus vector Ax-CM103G-ΔpreS (Suzuki, Saito, et. al., 2017, Vol. 7, pp. 41851) in which part of the S gene of the HBV is deleted. Three days after (day 0), when the cyclic HBV genome was replicating, the cells were co-infected at a moi of 7 each, with the eight guide expression vector designed to perform double-nicking cutting at four sites and with Cas9 nickase (NC9)- or Cas9-expressing adenovirus vector. On day 3 and day 7 after the co-infection, the whole DNA of the cells was extracted, and cyclic DNA was detected using the primers illustrated at the rightmost end of FIG. 11.

In FIG. 12, "cont" denotes a control infected with a so-called empty vector having no insert DNA, and the HBV genome produced by replicative HBV genome-producing adenovirus vector was replicated (2.8 kb, common in all the lanes (FIG. 12)). Meanwhile, when co-infection was performed using the eight guide expression vector, which performs double-nicking cuttings at four sites, and the Cas9 nickase expression vector, on day 7 after the infection, the amount of the uncut HBV genome (2.8 kb) generated from the replicative HBV genome-producing vector decreased about one fifth as compared with the control in which the HBV genome was freely replicating (FIG. 12, in the fourth and fifth lanes from the left without taking the marker (m) lane into consideration, comparison of the 2.8-kb band density on day 7 between cont and NC9). Meanwhile, deleted cyclic DNA that lost most of the HBV genome by double-nicking cuttings appeared (1.4 kb, the right-hand end in FIG. 11), and the amount thereof was much more than the amount of the 2.8 kb HBV genome during replication. The bands denoted by "*" are bands that non-specifically appear because they are observed in the controls as well. In this experimental system, the HBV genome continued to be always supplied from the replicative HBV genome-producing adenovirus vector, and thus the 2.8-kb band would not disappear. Moreover, almost no bands resulting from incomplete cutting was detected. Considering these, it was believed that cutting efficiency would be very high in the adenovirus vector designed to perform double-nicking cutting at four sites.

Another experiment in which the original Cas9 expression vector was used for co-infection instead of the Cas9 nickase expression vector was performed at the same time. In this case, simultaneous cutting of 4 pairs, 8 sites was performed by the original Cas9. However, its cutting efficiency was lower than in the case of performing double-nicking cutting at four sites (in the fifth and sixth lanes from the left without taking the marker (m) lane into consideration, comparison between NC9 and Cas9 on day 7). This result is considered to reflect the fact that the ends cut by the original Cas9 are blunt ends which are easily re-bonded to return to the original structure, whereas the ends of double-nicking cutting are hardly repaired as a result of formation of single-strand DNA having several tens of bases at the ends thereof.

Deletion of several tens of bases at the time of repair thereof can explain that the predicted 1.5 kb deleted DNA was detected in the original Cas9, whereas a shorter 1.4-kb band was detected in the double-nicking. The above result suggests that the eight guide expression adenovirus vector causing double-nicking cutting at four sites can have both high cutting efficiency and safety.

### [Example 6]

### Creation of adenovirus vector simultaneously expressing 12 guide RNA

In the same manner as in the creation of the adenovirus vector containing eight guide RNA expression units (see FIG. 7), creation of an adenovirus vector containing 12 guide RNA expression units (single guide RNA) was attempted. Guide coding DNA sequences used were eight sequences set forth in SEQ ID NOs. 5 to 12 mentioned above and the following four sequences set forth in SEQ ID NOs. 39 to 42 present downstream and in proximity to the stop codon of the X gene.
SEQ ID NO. 39 GCAGAGGTGAAAAAGTTGCA
SEQ ID NO. 40 GACATGAACATGAGATGATT
SEQ ID NO. 41 GCTGTGCCTTGGGTGGCTTT
SEQ ID NO. 42 GAAGCTCCAAATTCTTTATA

A cosmid used for creation of an adenovirus vector containing eight guide RNA expression units illustrated in FIG. 7 had a SgrDI cloning site having no other cutting sequence at the left-hand end of the eight guide RNA expression units thereof. Thus, four guide RNA expression units having guide coding DNA sequences set forth in SEQ ID NOs. 39 to 42 as mentioned above were inserted into this site. The obtained cosmid was used to study an adenovirus vector containing 12 guide RNA expression units.

As a result of studying separated six independent adenovirus vector clones, as illustrated in the left-hand figure of FIG. 13, five clones gave fragments (denoted by *) of the virus genome which were assumed to occur after deletion of several of the 12 units. However, one clone (clone 6, its lane is indicated with a box) gave almost no band indicating this deletion. The amount of the 5.5 kb fragment having the 12 units was slightly smaller than each of the amounts of the proximal bands slightly lower than 5 kb and derived from the vector backbone (these two bands slightly lower than 5 kb are indicated by two A). Thus, it was considered that this virus preparation stock involved almost no inclusion of vectors that deleted the units and most of them were vectors retaining the 12 units without deletion. That is, in the creation of the adenovirus vector having the 12 guide RNA expression units, differing from the case where the units were eight, most of the clone preparations were obviously recognized to involve inclusion of vectors having deletions. Surprisingly, however, it was possible to obtain a virus preparation stock involving a very low extent of inclusion of vectors having deletions in the creation of the adenovirus vector having the 12 guide RNA expression units.

This virus stock is the 2nd-generation passaged stock obtained by passaging the primary virus stock once to amplify the vector. Amplification was further performed by passaging to assess whether the 12 guide RNA expression units would be stably maintained in the 3rd-generation passaged stock and the 4th-generation passaged stock. The result is presented in the right-hand figure of FIG. 13. As a result, even in the 4th-generation passaged stock, the 5.5 kb fragment having the 12 units was observed as a clear band. The amount thereof was considered to be from about 1/2 to about 1/3 each of the proximal bands slightly lower than 5 kb and derived from the vector backbone. The present experiment just studied six clones. Studying more clones could be considered to make it possible to obtain a stock involving a much less extent of inclusion of deleted vectors.

### [Example 7]

Efficient disruption of HBV-X gene on chromosome by adenovirus vector

### simultaneously expressing 12 guide RNA

The 4th- or 5th-generation passaged stock is generally used in experiments using normal culture cells. This vector stock was infected under the same experiment conditions to the same cells as those in which the vector having the eight guide expression units illustrated in FIG. 9 and FIG. 10 had been used, to study whether all of the 12 guide RNA expressed from this vector would function using the X gene incorporated into the chromosomal DNA as the target. The newly added four guide RNA targets two sequences of the sequence of cut5 illustrated at the lowest row of FIG. 14 and the sequence of from the X gene to the MslI site at the end of DNA incorporated onto the chromosome (the other two target sequences of the guide RNA are outside the X gene and absent on the chromosome of this cell). The target sequence to the MslI site has one different base at the 5' end. It was therefore considered that no cutting would occur similar to the case where one base of the target sequence was different in the eight guide RNA expression units.

FIG. 15 illustrates the results of the above infection experiment using the adenovirus vector having the 12 guide RNA expression units. Similar to the experiment results using the adenovirus vector having the eight guide RNA expression units (FIG. 10), the 0.42-kb band indicating the deletion of from cut1 to cut4 was confirmed as expected. In addition, a 0.30-kb band was obviously observed, the band indicating the deletion of from cutting at cut5 to cutting at cut1 as expected in the newly added four guide expression units. Also, a 0.54-kb band occurring from un-cutting and the positions of two or more bands between the above 0.42-kb band and 0.30-kb band almost matched the positions of the bands occurring when cuttings occur at cut2 and cut3 at the same time as at cut1, cut4, and cut5. The above indicated that all of the guide RNA functioned that were expected to be cut by the adenovirus vector having the 12 guide RNA expression units.

### [Example 8]

### Creation of integration-type adenovirus vector containing DNA fragment encoding Cas protein and guide RNA expression units

Using the full-length DNA introducing method, attempt was made on creation of an adenovirus vector containing a DNA fragment encoding a Cas9 protein and four guide RNA expression units (single guide RNA). The guide coding DNA sequences used were identical to SEQ ID NOs. 1 to 4. Two kinds of adenovirus vector were obtained, where one is an adenovirus vector (inward) where the DNA fragment encoding a Cas9 protein is located upstream of the four guide RNA expression units and the other is an adenovirus vector (outward) where the DNA fragment encoding a Cas9 protein is located downstream of the four guide RNA expression units. The upper part of FIG. 16 presents the genome structure of the adenovirus vector, where the right-hand four rectangles denote the guide RNA expression units and the central, lower triangle denotes the deletion of the E3 region.

In the creation of an adenovirus vector using the full-length DNA introducing method, usually, a recombinant culture of 293 cells, which have been co-transduced with a cosmid at the final stage and a parental adenovirus vector, is cultured for 10 days after the transduction to obtain the target recombinant adenovirus vector.

This time, the target recombinant adenovirus vector could be obtained for the first time by continuing culturing for 20 to 24 days. In general, it is difficult to continuously culture 293 cells for 20 to 24 days. Further, production of an adenovirus vector containing only the DNA fragment encoding a Cas9 protein is not a simple process. In view of these, it is a surprising result that it was possible to obtain the adenovirus vector containing the DNA fragment encoding a Cas9 protein and the four guide RNA expression units.

As described above, the guide RNA expression units have common DNA sequences, and it is also an unexpected result that the four guide RNA expression units were retained after the long-term culturing of 20 to 24 days. The reason for this is unknown.

### [Example 9]

### Disruption in vivo of mouse H2Aa gene using integration-type adenovirus vector designed to percause double nicking at two site by four guide RNA

FIG. 17 illustrates the sequence of exon 1 of a mouse H2Aa gene that is the target gene to be knocked out. Exon 1 contains a 5' cap site and a start codon, and the figure indicates positions of four guide RNA target sequences (1 to 4) to perform two pairs of double-nicking cuttings (DN cuttings) set downstream thereof. The guide coding target sequences are SEQ ID NOs. 1 to 4 set forth above. The primer sequences used for PCR amplification are presented below.
Forward primer (F-primer)
   SEQ ID NO. 25 TTGGATCCAAGTCTGCAGCTGGCAACTTTGACG
Reverse primer (R-primer)
   SEQ ID NO. 26 AAAGAATTCTACACTACAGGTACAAAGGGCTTCAG

The above-produced integration-type adenovirus vector, having both the four guide RNA expression units (single guide RNA) targeting the mouse H2Aa gene and the Cas9 nickase expression unit, was purified and intravenously injected to neonatal mice, to study gene disruption efficiency in vivo and whether the four guides functioned through T7E1 assay (FIG. 18).

As a result, on day 7 after the administration of the vector, not only the 0.56-kb band indicating the unmodified genome fragment but also the band of about 0.33 kb and about 0.16 kb occurred after removal of the DN region with an enzyme, which were not observed in the control (administration of no vector, the second lane from the left without taking the marker lane into consideration (+ of Control)), were observed in all of the four mice (4 g out-m1, 4 g out-m2, 4 g in-m3, and 4 g in-m4). Also on day 15 after the administration of the vector, similar signals were observed (the lower row). In generally performed genome DNA cutting by a Cas9 enzyme and one guide RNA, the cut genome DNA forms minor insertions and deletions during repeating of end joining repairing and Cas9 cutting, and the reaction was terminated due to the resultant different sequences from the target 20-base sequence. T7E1 assay forms two bands by cutting a branched portion between single-stranded DNA and double-stranded DNA occurring as a result of thermal deformation of PCR fragments, which are double-stranded DNA, followed by pairing. Thus, the sizes of these two bands are only slightly different from the size of the uncut fragment. However, the inventors' experiments have revealed that in double nicking by Cas9 nickase, one-site cutting causes deletion of several tens of bases having random lengths in the target DNA site. The resultant DNA fragments are considered to lead to wide bands ranging from the upstream end to around the random DN cutting about 160-base downstream by guide 1 and guide 2 and ranging from around the random DN cutting to the downstream end about 330-base downstream by guide 3 and guide 4. Specifically, as a result of deletion and removal of the DNA between the two DN cuttings, the difference between these two band sizes is considered to indicate the size of the region removed. In the present experiment, the expected bands of 0.33 kb and 0.16 kb were observed, indicating that about 70 bases between the two DN cuttings were deleted and therefore all of the four guide RNA functioned. In the lanes of all of the eight mice, the deletion efficiency was found to be 17% and 19%. It was considered from these results that the four guide expression integration-type vector caused double-nicking cutting.

### [Example 10]

### Creation of integration-type adenovirus vector having eight guide RNA expression units and Cas9 nickase expression units

The upper limit of the size of the adenovirus genome packaged in a virus particle is 38.0 kb, and the size of the vector genome whose E3 region had been shortened was found to be 30.2 kb. Thus, foreign DNA that can be incorporated is up to about 7.8 kb. The expression unit of the Cas9 nickase is 5.3 kb, and thus the length that can be used for a multiple guide expression unit is up to 2.5 kb. Meanwhile, the size of one normal guide expression unit is about 0.4 kb, and thus the length of eight units is 3.2 kb, which considerably exceeds 2.5 kb. A normal expression unit cannot be used for the creation.

In view thereof, attempt was made on shortening a U6 promoter in the expression unit. FIG. 19 illustrates the structure of the guide RNA expression unit. The U6 promoter has two essential elements (DSE and PSE) and is known in basic studies to retain its activity even after deletion of the intermediate region between them (e.g., Stunlel et al., Molec. Cell. Biol., 1997, vol. 17, No. 8, pp. 4397-4405). However, the size of the U6 promoter is small enough for uses of normal purposes. The U6 promoter having the intermediate region deleted is hardly used in applied studies. The PCR primers illustrated below and in FIG. 19 were used for a plasmid having the normal U6 promoter, to prepare a fragment that did not include 135 bases corresponding to the intermediate region between the DSE and PSE elements and did include the whole portion of the plasmid except for these. Next, this fragment was cut with BsrGI and further treated with a Klenow enzyme for blunting, followed by self-ligation, to obtain a plasmid having a shortened U6 promoter having a SnaBI sequence (TGTACGTACA) as a binding end. In FIG. 19, the deleted potion is indicated by a blank arrow. Also, six bases at the 3' end of the U6 promoter (positions of from -6 to -1) were made as a BamHI recognition sequence. The base sequences of the primers used are given below.
Reverse primer containing the DSE sequence
   SEQ ID NO. 27 GCGTGTACAGTATATGCAAATATGAAGGAATCATGGG
Forward primer containing part of the PSE sequence
   SEQ ID NO. 28 GCGTGTACAAAATGGACTATCATATGCTTACCGTAAC

As a result, the size of each guide RNA expression unit was shortened from 0.4 kb to about 0.28 kb, and the length of the eight guide expression units was about 2.3 kb, which was slightly below 2.5 kb; i.e., the upper limit of the size that can be incorporated into the vector. This led to the possibility for the capability of creating an integration-type vector having eight guide RNA expression units.

FIG. 20 illustrates the sequence of exon 2 in a mouse NTCP gene that is the target gene to be knocked out. Exon 2 contains a start codon and eight guide RNA target sequences (1 to 8) to perform four pairs of double-nicking cuttings (DN cuttings) set downstream thereof. In the present experiment, four-site, double-nicking cuttings were proximally set within one exon. However, it is also possible to set them so as to perform disruption by two-site cutting of two genes or simultaneous disruption of four different genes. The following are the sequences of the target 20 bases used for the creation of the eight guide RNA expression units having the above target sequence.
SEQ ID NO. 29 GCGGCAGGGAGAAATTGAAG
SEQ ID NO. 30 GCCGCCTGGCTTTGGCCACC
SEQ ID NO. 31 GATGAAAGACCTTGCCCAGA
SEQ ID NO. 32 GCTCTGGCCATCCTCATCTG
SEQ ID NO. 33 GATACCGTACTGGGCCACTA
SEQ ID NO. 34 GCCCCTCAGTGCTTTCCTTC
SEQ ID NO. 35 GAGGTTCATGTCCCCCTTCA
SEQ ID NO. 36 GGACCGAGGGTTAAGAGGAA

An integration-type adenovirus vector (the upper part of FIG. 21) was created that has the eight guide RNA expression units having the above shortened U6 promoter and Cas9 nickase expression units. The vector genome full-length DNA was transfected into 293 cells to obtain a virus vector. A period required for a virus to amplify to obtain a clone is usually two weeks, but three weeks was required similar to the four guide expression integration-type vector. In the four guide integration-type, all of the clones had no deletion and retained the four guide units, while in the eight guide integration-type, most of the six clones were observed about deletion of the units (the left-hand figure of the lower part of FIG. 21) but the clone amplified without any deletion could be obtained [No. 6; the 3.0-kb band contains the eight guide expression units, and the other clones gave the bands indicated by * and containing the deleted units). This virus clone was passaged to prepare a purified virus in such an amount as to be able to perform animal experiments. As a result, most of the virus DNA retained the eight units (the lower, right-hand figure in FIG. 21; the 3.0-kb band), suggesting that an eight guide RNA simultaneous expression experiment would be possible. To date, three kinds of the eight guide RNA expression integration-type vectors were successfully created, and they gave almost similar results.

### [Example 11]

### Disruption in vitro of mouse NTCP gene using integration-type adenovirus vector designed to perform four-site simultaneous cuttings by double-nicking method

Studies were conducted for disruption of the target gene by an integration-type adenovirus vector designed to perform four-site, double-nicking cuttings. An adenovirus is a virus that infects humans, and as illustrated in FIG. 9, the disruption efficiency of the HBV gene on the chromosome of a human-derived culture cell line (HepG2) was found to be about 90%. The target this time is a mouse gene, which needs an experiment in mouse culture cells. The introduction efficiency of the adenovirus vector into the mouse cells is about 1/100 or lower than in human cells, which makes experiments difficult. Nevertheless, when this vector of an amount 30 times that in the experiment of FIG. 9 was infected to primary mouse embryonic fibroblasts (MEF cells), the gene deletion disruption efficiency of 56% was achieved (the left-hand figure in FIG. 22). Also, the deletion of about 150 bases suggested highly efficient deletion by simultaneous cuttings of guide RNA 1 and 2 and guide RNA 3 and 4 or highly efficient deletion by simultaneous cuttings of guide RNA 3 and 4 and guide RNA 7 and 8 (see FIG. 20). One deletion indicates that four guide RNA simultaneously functioned to make nicks. Thus, the indicated one deletion by two or more double-nicking cuttings is considered to suggest the ability to disrupt a gene efficiently and safely. Also, at the lanes of virus loads (moi) of 100 and 300, short DNA regions of several tens of bases (indicated by *) were observed just below the 6.5-kb band of the full-length PCR fragment. The inventors' experiments, however, revealed that cutting of one site by double nicking caused deletion of several tens of bases. This region suggests that gene disruption was performed by not only large deletion by simultaneous cuttings at two or more sites but also by short deletion at four sites or several individual cutting sites.

At the virus loads of 100 and 300, a smear region was observed at a position of 0.5 kb. This smear region was indicated to be denser as the infected virus load increased in the photograph of the same electrophoresis gel under long-term exposure (0.5 in the right-hand figure in FIG. 22). Also, at the infected virus loads of 100 and 300, 0.3-kb regions were observed. In the present PCR, no occurrence of deletion results in formation of a 0.65-kb band. The 0.5-kb and 0.3-kb regions are considered to occur as a result of deletions of about 150 bases and about 350 bases, respectively. These lengths matched the expected deletion between guides 1 and 2 and guides 3 and 4, the expected deletion between guides 3 and 4 and guides 7 and 8, and the expected deletion between guides 1 and 2 and guides 7 and 8 (indicated by the three dotted lines in FIG. 20). That is, it was considered that the eight guides almost simultaneously functioned to cause deletion. The above results indicate that the eight guide units-containing integration-type adenovirus vector designed to perform double-nicking cuttings at four sites can perform simultaneous cutting at two or more sites even in mouse cells having low infection efficiency.

### [Example 12]

### Disruption in vivo of mouse NTCP gene using integration-type adenovirus vector designed to percause double nicking cuttings at four sites by eight guide RNA

The above integration-type adenovirus vector designed to percause double nicking cuttings at four sites by eight guide RNA was intravenously injected to neonatal mice, to perform a disruption experiment in vivo of a mouse NTCP gene. The results are presented in FIG. 23. On day 15, 1.05-kb and 0.85-kb bands were observed in all of the second mice (8g m2), the third mouse (8g m3), and the fourth mouse (8g m4). It was considered from this result that many of the eight guide RNA functioned to cause deletion. The base sequence of the primers used are given below.
Primer mNTCPint1-BbsI
   SEQ ID NO. 37
   GTCATGGACACAGCCTCTCCTGAAGACAGC
Primer mNTCPint2-AlwI
   SEQ ID NO. 38
   CCTTTACAGTGATCCTGTAGAGACTTCTGG

### [Example 13]

### Creation of lentivirus vector having eight guide RNA expression units (single guide RNA) designed to perform double-nicking cuttings at four sites

A lentivirus vector pLVSIN-EF1αPur (obtained from Takara Bio Inc.), having an EF1α promoter to express the target gene and expressing puromycin (Pur) was cut with EcoRI and Sbfl and treated with a Klenow enzyme for blunting, followed by binding, to obtain plasmid pLVSINwPur in which the EF1α promoter region was deleted. This was cut with SgrDI, and a lentivirus vector fragment having a blunt end formed by the Klenow enzyme and a NheI end was cloned between SmaI and XbaI of charomid 9-36 (Saito et al., Proc. Natl. Acad. Sci., 1996, vol. 83, pp. 8664-8668), to obtain cosmid chLVpur-w. This cosmid was cut with BstXI and treated with a Klenow enzyme into a ring, to obtain 40.1-kb cosmid cassette chLVpurkx-w having no BstXI site. Similar to the cosmid for preparing the adenovirus vector having the eight guide expression units, by incorporating a multiple guide RNA expression unit fragment into a SwaI cloning site of the lentivirus vector, this cosmid was amplified, using an in vitro packaging of λ phage, in Escherichia coli. without deletion of the multiple guide RNA expression unit fragment.

The below-described fragment containing eight guide RNA expression units containing guide coding DNA sequences in eight guide RNA designed to perform double-nicking cuttings at four sites to target the X gene was prepared to obtain 43.2-kb cosmid chLVpurk-g8DNHBxKK incorporated into the SwaI site of chLVpurkx-w. This cosmid has unnecessary spacer sequence of about 30 kb and thus is not suitable for obtaining a lentivirus vector through transfection. In view thereof, the spacer was removed with PhsAI that cuts the spacer sequence. Then, a 43.2-kb triangular cosmid having the obtained three 14.4-kb fragments linked together, each of the fragments contains the eight guide RNA expression units and the lentivirus vector, was created by the method of Nakanishi et al. (Nakanishi et al., J. Gene Med., 2019, e3115). This cosmid can be amplified in Escherichia coli. without deletion of the multiple guide RNA expression unit. This DNA was cut with ApaI to be linear and then was transfected to 293T cells, to obtain lentivirus vector LVpur-g8DNHBxKK.

FIG. 24 illustrates the positions of the guide RNA target sequences on the X gene. The guide coding DNA sequences in eight guide RNA used for double nicking are given below.
SEQ ID NO. 43 AGACAAAGGACGTCCCGCGC
SEQ ID NO. 44 GACCCGTCTCGGGGCCGTTT
SEQ ID NO. 45 GCCGGAACGGCAGATGAAGA
SEQ ID NO. 46 GGGGCGCACCTCTCTTTACG
SEQ ID NO. 47 GGTCTCCATGCGACGTGCAG
SEQ ID NO. 48 GACCACCGTGAACGCCCACC
SEQ ID NO. 49 GTCCTCTTATGTAAGACCTT
SEQ ID NO. 50 GATGTCAACGACCGACCTTG

A forward primer used for PCR for studying deletion was that set forth in the following SEQ ID NO. 51 present in a Neo gene used for introducing X gene expression units into the chromosome upstream the X gene.
Forward primer:
SEQ ID NO. 51 ATCTTATCATGTCTGGATCAAATCCGAACGC

A reverse primer used was the primer set forth in SEQ ID NO. 14 used for amplifying the X gene in Example 4.

Use of these two primers generates a 0.66-kb fragment containing the X gene for performing double-nicking cuttings in four different ways. However, the 5' end of the guide RNA set forth in SEQ ID NO. 43 and generated by the U6 promoter (guide RNA indicated by "1" in FIG. 24) is A, and the 5' end of RNA generated from the U6 promoter is optimally G. Thus, the amount of this guide RNA produced was expected to be small. As a result, the cutting efficiency of double nicking with this guide RNA in one of them was expected to be low.

The lentivirus vector incorporated into the cell chromosomal DNA expresses eight guide RNA. It was considered that when Cas9 or Cas9 nickase was supplied thereto by an adenovirus vector, they recognized the target sequences on the X gene DNA on the chromosome to cause cutting at eight sites (Cas9) or double-nicking cuttings at four sites (Cas9 nickase) (FIG. 24). A culture supernatant of the 293T cells containing the lentivirus vector was infected to GX11 cells having the X gene on the chromosome. Six days later, the GX11 cells were infected with an adenovirus vector expressing Cas9 or Cas9 nickase (denoted by Cas9 or NC9) or a control vector having no expression unit (denoted by 1w1) at a moi of 5. Experiment results are presented in FIG. 25.

FIG. 25 is a view illustrating the results obtained after six days from the infection with the adenovirus vector expressing Cas9 or Cas9 nickase (denoted by Cas9 or NC9) or a control vector having no expression unit (denoted by 1w1). In the case of infecting the Cas9-expressing adenovirus vector (the lane is denoted by Cas9) in the cells having on the chromosome the lentivirus vector having the X gene of HBV and the eight guide RNA expression units, such bands were detected that were formed by two or more cuttings of the 0.66-kb full-length fragment obtained through PCR. It was considered from their sizes that the 0.58-kb band was formed through simultaneous cutting by "1" guide RNA and "3" guide RNA or "5" guide RNA and "8" guide RNA. Similarly, it was considered that the 0.48-kb band was formed through simultaneous cutting by "1" guide RNA and "5" guide RNA or "3" guide RNA and "8" guide RNA, and the 0.38-kb band was formed through simultaneous cutting by "1" guide RNA and "8" guide RNA. Thus, it was indicated that two or more guide RNA including at least "1" guide RNA and "8" guide RNA among the eight guide RNA functioned. Meanwhile, in the case of the Cas9 nickase-expressing adenovector (lane NC9), a smear region was seen under the 0.66-kb full-length fragment, especially from 0.60 kb to 0.55 kb, suggesting that two or more double-nicking cuttings occurred as a result of deletions of various sizes by two or more double nicking.

From the results of the present experiment, the cutting efficiency of the target gene does not seem to be high, but is not necessarily so. In usual experiments using a lentivirus vector, a puromycin-resistant gene is incorporated into a vector to express it, and only the cells whose chromosomes incorporate the lentivirus vector are selected. The lentivirus vector used in the present experiment is also allowed to express a puromycin-resistant enzyme, but puromycin selection is not performed in this experiment. It is therefore considered that the efficiency seems to be very low because most of the cells do not have this lentivirus vector. In general experiments involving puromycin selection, therefore, the gene cutting disruption efficiency is considered to be much higher than the results of the present experiment.

## Claims

1. A recombinant adenovirus vector,
wherein a region having a length of more than 2685 base pairs lacks from an E3 region.

2. The recombinant adenovirus vector according to claim 1, wherein a region of 2837 base pairs lacks from the E3 region.

3. The recombinant adenovirus vector according to claim 1, wherein the recombinant adenovirus vector is an Ad5 vector-derived recombinant adenovirus vector where 27982^{th} to 30818^{th} bases as positions in an Ad5 vector are deleted from the E3 region.

4. A nucleic acid vector, which expresses five or more Cas guide RNA.

5. The nucleic acid vector according to claim 4, wherein the nucleic acid vector expresses eight Cas guide RNA.

6. The nucleic acid vector according to claim 5, wherein the nucleic acid vector is configured to introduce at least four double nicking to cause at least 4 genome cuttings.

7. The nucleic acid vector according to claim 6, wherein the nucleic acid vector is designed to knock down a target sequence to be knocked down on a genome by introducing two double nicking into a region upstream and in proximity to the target sequence and further introducing two double nicking into a region downstream and in proximity to the target sequence.

8. The nucleic acid vector according to any one of claims 4 to 7, wherein the nucleic acid vector is the recombinant adenovirus vector according to any one of claims 1 to 3.

9. A nucleic acid vector, which is an integration-type Cas nucleic acid vector including a Cas protein-coding gene and a Cas guide RNA expression unit.

10. The nucleic acid vector according to claim 9, wherein the nucleic acid vector includes three or more Cas guide RNA expression units.

11. The nucleic acid vector according to claim 10, wherein the nucleic acid vector includes four Cas guide RNA expression units.

12. The nucleic acid vector according to any one of claims 9 to 11, wherein at least one pair of the Cas guide RNA expression units is configured to cause double nicking.

13. The nucleic acid vector according to any one of claims 9 to 12, wherein the nucleic acid vector is the vector according to any one of claims 1 to 8.

14. A nucleic acid vector comprising:
a Cas guide RNA expression unit;
a Cas protein-coding gene; and
a donor DNA sequence to be knocked in.

15. The nucleic acid vector according to claim 14, wherein the nucleic acid vector includes two or more Cas guide RNA expression units, which are configured to introduce double nicking.

16. The nucleic acid vector according to claim 14, wherein the nucleic acid vector includes eight Cas guide RNA expression units, which are configured to introduce four double nicking.

17. The nucleic acid vector according to any one of claims 14 to 16, wherein the nucleic acid vector is the vector according to any one of clams 1 to 13.
